# EUROPEAN PATENT SPECIFICATION

(11) **EP 4 390 848 B1**
(45) Date of publication and mention of the grant of the patent: **09.12.2026**
(21) Application number: 22215959.2
(22) Date of filing: 22.12.2022
(51) Int. Cl.: G06T 7/73

(54) **IMPROVED METHOD FOR TRAINING A POSE ESTIMATOR**
VERBESSERTES VERFAHREN ZUM TRAINIEREN EINES HALTUNGSSCHÄTZERS
PROCÉDÉ AMÉLIORÉ D'APPRENTISSAGE D'UN ESTIMATEUR DE POSE

(43) Date of publication of application: 26.06.2024
(73) Proprietor: Robert Bosch GmbH, 70442 Stuttgart (DE)
(72) Inventor: Leibe, Bastian, 52134 Herzogenrath (DE); Linder, Timm, 71032 Boeblingen (DE); Hermans, Alexander, 52068 Aachen (DE); Sárándi, István, 52074 Aachen (DE)

(56) References cited:
- XU JIACHEN ET AL: "Exploring Versatile Prior for Human Motion via Motion Frequency Guidance", 2021 INTERNATIONAL CONFERENCE ON 3D VISION (3DV), IEEE, 1 December 2021 (2021-12-01), pages 606 - 616, XP033999135, DOI: 10.1109/3DV53792.2021.00070
- WENTAO ZHU ET AL: "MotionBERT: Unified Pretraining for Human Motion Analysis", ARXIV.ORG, CORNELL UNIVERSITY LIBRARY, 201 OLIN LIBRARY CORNELL UNIVERSITY ITHACA, NY 14853, 12 October 2022 (2022-10-12), XP091342487

## Description

### TECHNICAL FIELD

The presently disclosed subject matter relates to a method for training a pose estimator, a method for controlling a robotic system, a system, and a computer readable medium.

### BACKGROUND

A robust 3D scene understanding, including the ability to accurately detect and localize both objects and humans as well as their, possibly articulated, poses in 3D space, is a fundamental capability for an autonomous mobile system to take safe, efficient, and informed actions especially in challenging cluttered and populated environments. In the past decade, different methods have been developed to estimate the poses of objects and humans using, e.g., key point-based techniques.

For objects, key points of interest may for example include the corners of a bounding box, and the derived object poses can enable, for instance, robotic manipulation tasks. For perceiving and understanding humans, it can be helpful to estimate not only the overall position of their torsos relative to the sensor, but to estimate their fully articulated poses including the positions of all body joints, represented as key points. This can for example enable an autonomous system to classify the current action and activities a human is pursuing, and to anticipate future human actions, or enable gesture-based interactions with the system.

Jiachen Xu et al., "Exploring Versatile Prior for Human Motion via Motion Frequency Guidance", 2021 International Conference on 3D Vision (3DV), IEEE, 1 December 2021, pages 606 - 616, DOI: 10.1109/3DV53792.2021.00070 discloses human motion tracking for different goals independent of the person's orientation using a trained variational autoencoder.

Wentao Zhu et al., "MotionBERT: Unified Pretraining for Human Motion Analysis", ARXIV.ORG, Cornell University Llibrary, Ithaca, NY 14853, 12 October 2022 discloses human motion analysis from skeletal joints for various downstream tasks.

However, current techniques for pose estimation do not yet yield sufficiently robust results in absolute, metric 3D space especially under heavy occlusion, and require very large datasets to be combined for training that often follow heterogeneous key point definitions or skeleton formats which may result from different ground truth acquisition techniques and labeling strategies. Likewise, approaches for human action and activity recognition and anticipation, which may depend on the output of a human pose estimator, require large domain-specific training datasets and do not easily transfer towards new scenarios that are relevant, e.g., for robotics or automotive applications. Deep learning-based 3D pose estimation such as human pose estimation performs best when trained on large amounts of labeled data. However, obtaining large datasets is resource intensive. Combining existing datasets is not straightforward, because of the different pose formats, or skeleton formats, provided by different datasets, e.g., they do not label the same set of key points, e.g., anatomical landmarks.

### SUMMARY

It would be desirable to have an improved method for training a pose estimator. A pose estimator may be configured to receive as input a sensor measurement representing an object and to produce a pose, a pose comprising a plurality of key points identified in the object determining a pose of the object.

The invention is defined by the independent claims; the dependent claims provide advantageous embodiments.

Poses may be estimated; for example, for the task of a generic object pose or articulated human body pose estimation, e.g., for the purpose of localizing e.g., the corners of one or multiple objects or the body joint positions of one or multiple humans, given a sensor measurement. A pose is a set of key points identified for the object. A pose is typically a set of points in a 3D space, although a pose could be a 2D pose. A pose is sometimes referred to as a skeleton. Key points are sometimes referred to as landmarks. In addition to a key point, a pose may include further information. For example, the orientation of a joint, or a wheel or the like, may be included in the pose. A pose estimator may be applied to objects, other than humans, which are representable by key points, e.g., to estimate the pose of a car or bicycle in traffic that shall be avoided by a control unit, or for object pose estimation in robotic manipulation tasks, such that a control unit can plan and execute a trajectory for grasping an object. A pose may be used to determine the orientation of an object with respect to another object, e.g., with respect to the sensor, a vehicle, or the like.

For example, a pose for a vehicle may comprise key points of the vehicle that indicate an orientation of the vehicle, and/or an orientation of key parts, e.g., the wheels.

The way a 3D object is mapped to a pose, is referred to as the pose format. For example, the pose format may be a convention, which key points to include, what order to include them, and where or how to measure them. Typically, two different training data sets use different pose formats, making the training data sets incompatible. A pose format is also referred to as the key point definitions, skeleton format, or skeleton convention.

For example, a pose format could define a human skeleton with a left/right shoulder, elbow and wrist joint as well as a neck joint, which are connected to each other through bones. A pose format can be defined for all kinds of articulated and non-articulated objects, e.g., humans, animals, the corners of a box, vehicles, or a pallet truck with a movable handle. Vehicles include, e.g., cars, trucks, bicycles, and forklifts.

In the past, attempts have been made to translate the poses of one training data set to the pose format of a second training data set by applying a set of handcrafted rules. This approach introduces inaccuracies, and is time-consuming if the number of training data sets is large. The problem becomes more severe, the more datasets with different skeletons are combined during training. Embodiments have been applied to many training data sets, e.g., ten or more, and twenty or more. This is intractable for handcrafted rules.

Different pose formats can result from e.g., different technologies and protocols being used during dataset acquisition, e.g., motion capture/marker-based approaches vs. marker-free approaches, synthetically rendered training data, output of commercially available body tracking systems, changes in labeling conventions, etc. The skeletons may differ in their number, location, and meaning of body joints, e.g., the "left knee" joint on one human skeleton may reside inside the knee, whereas on another skeleton that was recorded e.g., using reflective markers in a motion capture setup, the corresponding joint may be located 5 cm towards the front, on top of the knee cap. Likewise for more generic objects, e.g., some datasets may have labels for corner key points, while others may use other landmarks of an object as key points.

For example, an embodiment of a method for training a pose estimator may obtain multiple trained initial pose estimators, trained on multiple training data sets. A pose estimator may not be trainable on all or even more than one of the training data sets, as the multiple training data sets may use a different pose format. It is not straightforward to translate from one pose format to another. For example, the pose format may only be implicitly known from the training data set. A pose format is typically not well-defined.

Having the trained initial pose estimators, an embodiment may comprise training an autoencoder. For example, the autoencoder may be configured and trained to receive at least one pose according to a first pose format corresponding to a first training data set and to produce at least one pose according to a second pose format corresponding to a second training data set. Training the autoencoder may be done on a pool, e.g., a set of sensor measurements and multiple estimated poses for the sensor measurements in the pool. For example, the multiple initial pose estimators may be applied to the sensor measurement in the pool; multiple poses according to different pose formats are thus obtained for the same sensor measurement.

Having the autoencoder, a further pose estimator may be trained using more than one of the multiple training data sets using at least part of the trained autoencoder to map a pose from a pose format corresponding to a first training data set to a pose format corresponding to a second training data set. Training the further pose estimator may not need the entire autoencoder, only, e.g., a decoder part thereof. After training the further pose estimator, the further pose estimator may be restricted to only predict poses according to a preferred pose format. At inference time, part of the autoencoder, e.g., the decoder part, may remain part of the deployed network, although this is not necessary.

The resulting further pose estimator after training yields more consistent and more accurate pose estimates, e.g., because more training data is available for training the further pose estimator, and because additionally introduced loss functions for autoencoder-based regularization interrelate the different pose formats with each other to make the further pose estimator output more consistent. Experiments showed that on a variety of benchmarks, embodiments of the methods yields improved results.

Typically, both the initial pose estimators as well as the further pose estimator comprises a neural network, or even a system of connected neural networks. This is not necessary though; embodiments can be adapted to any pose estimation method used for the initial pose estimators, while the further pose estimator is at least trainable, e.g., machine learnable.

Typically, the further pose estimator uses the same or similar technologies as the initial pose estimators, or as one of the initial pose estimators; For example, both may comprise a neural network, for example, both may comprise a neural network of the same size. This is not necessary though. For example, the initial pose estimators may use a variety of technologies, some or all of which may be different from the further pose estimator. For example, the further pose estimator may be larger than the initial pose estimators, e.g., comprise more parameters, e.g., comprise a larger neural network.

An embodiment of the method of training a pose estimator may provide an automatic, learning-based approach to map different poses with different pose formats to each other. In an embodiment, this mapping is through a latent key point representation. For example, a dimensionality reduction technique may be applied to the key points in a pose. Interestingly, points in the latent space may be defined as linear combination of points in a pose. It was found that affine combination works particularly well, as they are invariant under space transformation, are performant, and require few resources. Pose estimation may be done from a variety of sensor measurements. The sensor measurements provide information on the pose of an object. Typically, the sensor measurements are multidimensional, e.g., at least two-dimensional. For example, a sensor measurement may be an image. For example, the sensor measurement may be obtained from a camera, e.g., a visible light camera, infrared camera, etc. The sensor measurement may be a monocular image, a polyocular image, e.g., binocular image, e.g., comprise two images of the same object from different viewpoints, or multi-ocular, e.g., comprise multiple images of the same object from multiple different viewpoints, e.g., more than 2. A sensor measurement may also be a distance-based measurement, e.g., a range image or a point cloud obtained from a LIDAR, or a depth image from a time-of-flight camera, or a radar measurement. It may also be a combination of multiple of the aforementioned sensor modalities, e.g., comprising a color image and a depth image, or an infrared image and a point cloud.

A further aspect concerns a method for controlling a robotic system. The method may comprise obtaining a further pose estimator trained according to an embodiment. For example, the method may comprise receiving the trained further pose estimator, or may comprise training the further pose estimator. The method may comprise applying the further pose estimator to a sensor measurement and obtaining an estimated pose. A control signal may be derived from at least the pose. The signal may be transferred to control the robotic system. For example, a human pose may be estimated, from which it may be derived that the human is about cross the road, so that a braking control signal is derived and transferred, e.g., to actuators of an autonomous vehicle. Examples of robotic systems include, autonomous vehicles, robotic arms, autonomous manufacturing, etc.

Aspects include a method for training a pose estimator, a method of estimating a pose, a system for training a pose estimator, a system for estimating a pose. The systems are electronic systems, typically, digital systems. The systems may comprise a computer. A system may comprise: one or more processors; and one or more storage devices storing instructions that, when executed by the one or more processors, cause the one or more processors to perform operations for a method according to any one of the preceding claims.

The method of training a pose estimator, and the resulting trained pose estimator may be applied in a wide range of practical applications. Such practical applications include autonomous vehicle control, robotic system control, and so on.

For example, an embodiment of a method of training a further pose estimator may comprise applying multiple trained initial pose estimators to a pool of sensor measurements to obtain multiple estimated poses for the sensor measurements in the pool. A further pose estimator may be trained on multiple training data sets using at least part of an autoencoder to map a pose from a first pose format to a second pose format. The autoencoder may be trained on the multiple estimated poses.

An embodiment of a method of training a pose estimator, a method of pose estimation, and method of controlling a robotic system, may be implemented on a computer as a computer implemented method, or in dedicated hardware, or in a combination of both. Executable code for an embodiment of the method may be stored on a computer program product. Examples of computer program products include memory devices, optical storage devices, integrated circuits, servers, online software, etc. Preferably, the computer program product comprises non-transitory program code stored on a computer readable medium for performing an embodiment of the method when said program product is executed on a computer.

In an embodiment, the computer program comprises computer program code adapted to perform all or part of the steps of an embodiment of the method when the computer program is run on a computer. Preferably, the computer program is embodied on a computer readable medium.

Another aspect of the presently disclosed subject matter is a method of making the computer program available for downloading.

### BRIEF DESCRIPTION OF DRAWINGS

Further details, aspects, and embodiments will be described, by way of example only, with reference to the drawings. Elements in the figures are illustrated for simplicity and clarity and have not necessarily been drawn to scale. In the figures, elements which correspond to elements already described may have the same reference numerals. In the drawings,
Figure 1.1 schematically shows an example of an embodiment of a pose estimator training device,
Figure 1.2 schematically shows an example of an embodiment of a pose estimator,
Figure 1.3 schematically shows an example of an embodiment of a robotic system,
Figure 2.1 schematically shows an example of an embodiment of multiple training data sets and multiple initial pose estimators,
Figure 2.2 schematically shows an example of an embodiment of multiple training data sets, a shared backbone and multiple prediction heads,
Figure 2.3 schematically shows an example of an embodiment of a pool of sensor measurements,
Figure 3 schematically shows an example of an embodiment of autoencoder training data,
Figure 4.1 schematically shows an example of an embodiment of an autoencoder,
Figure 4.2 schematically shows an example of an embodiment of an autoencoder,
Figure 5 schematically shows an example of an embodiment of pose estimators,
figure 6.1 schematically shows an example of an embodiment of pose estimators,
Figure 6.2 schematically shows an example of an embodiment of pose estimators,
Figure 6.3 schematically shows an example of an embodiment of pose estimators,
Figure 7.1 schematically shows a line drawing of an example of an embodiment of a sensor measurement,
Figure 7.2 schematically shows an example of an embodiment of initial pose estimations in a front view,
Figure 7.3 schematically shows an example of an embodiment of initial pose estimations in a side view,
Figure 7.4 schematically shows an example of an embodiment of pose estimations in a front view,
Figure 7.5 schematically shows an example of an embodiment of pose estimations in a side view,
Figure 8.1 schematically shows an example of an embodiment of a method for training a pose estimator,
Figure 8.2 schematically shows an example of an embodiment of a method for controlling a robotic system,
Figure 9.1 schematically shows a computer readable medium having a writable part comprising a computer program according to an embodiment,
Figure 9.2 schematically shows a representation of a processor system according to an embodiment.

### Reference signs list

The following list of references and abbreviations corresponds to figures 1.1-6.3, and 9.1-9.2, and is provided for facilitating the interpretation of the drawings and shall not be construed as limiting the claims.
- 10: a system
- 11: a pose estimator training device
- 13: a processor system
- 14: storage
- 15: communication interface
- 31: a pose estimator device
- 33: a processor system
- 34: a storage
- 35: a communication interface
- 40: a robotic system
- 41: a sensor
- 42: a controller
- 43: a control signal
- 44: an actuator

- 210, 220, 230: training data set
- 211, 221, 231: sensor measurements
- 212, 222, 232: poses
- 213, 223, 233: initial pose estimators
- 214, 224, 2234: training initial pose estimators
- 300: shared backbone
- 313, 323, 333: prediction head
- 240: a pool of sensor measurements
- 241-243: a sensor measurement
- 215, 225, 235: applying initial pose estimators
- 250.1-250.3: set of estimated poses
- 251.1-253.3: estimated pose
- 260: autoencoder training data
- 260.1-260.3: autoencoder training data element
- 400: an autoencoder
- 410: an encoder
- 401: a latent space
- 420: a decoder
- 430: an autoencoder
- 216, 226, 236: applying pose estimators
- 211.1: a sensor measurement
- 217, 227, 237: an estimated pose
- 218, 228, 238: a reconstructed estimated pose
- 212.1: a pose
- 271, 272: a pose loss
- 273: a consistency loss
- 311: a sensor measurement
- 320, 330: a pose estimator
- 321, 322: estimated poses
- 323: an estimated pose in latent space
- 402: a teacher loss
- 1000, 1001: a computer readable medium
- 1010: a writable part
- 1020: a computer program
- 1110: integrated circuit(s)
- 1120: a processing unit
- 1122: a memory
- 1124: a dedicated integrated circuit
- 1126: a communication element
- 1130: an interconnect
- 1140: a processor system

### DESCRIPTION OF EMBODIMENTS

While the presently disclosed subject matter is susceptible of embodiment in many different forms, there are shown in the drawings and will herein be described in detail one or more specific embodiments, with the understanding that the present disclosure is to be considered as exemplary of the principles of the presently disclosed subject matter and not intended to limit it to the specific embodiments shown and described. In the following, for the sake of understanding, elements of embodiments are described in operation. However, it will be apparent that the respective elements are arranged to perform the functions being described as performed by them.

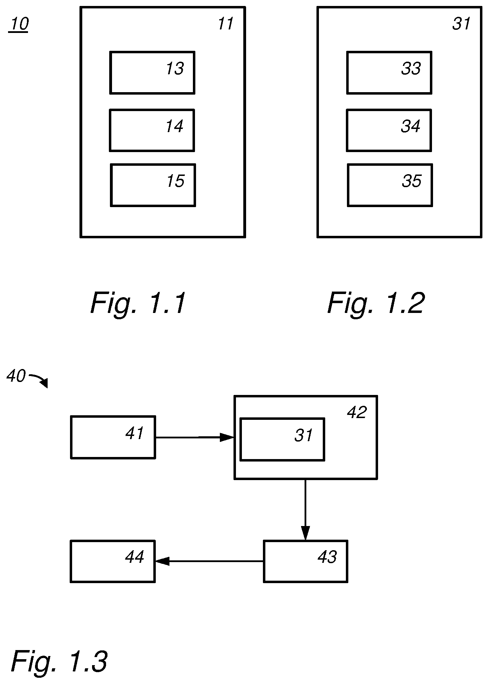

**Figure 1.1** schematically shows an example of an embodiment of a pose estimator training device 11. **Figure 1****.****2** schematically shows an example of an embodiment of a pose estimator device 31. Pose estimator training device 11 and pose estimator device 31 may be part of a system 10.

The pose estimator device 31 is configured to receive as input a sensor measurement representing an object and to produce a pose for the object. Pose estimator training device 11 is configured to train pose estimator device 31 using multiple training data sets, which may not have a common pose format. For example, the system 10 may be used to train a pose estimator to a higher accuracy than is obtained from using fewer training data sets. The trained pose estimator may be used to control autonomous systems, e.g., vehicles, robotics systems, and the like.

Pose estimator training device 11 may comprise a processor system 13, a storage 14, and a communication interface 15. Pose estimator device 31 may comprise a processor system 33, a storage 34, and a communication interface 35. Storage 14 and 34 may be, e.g., electronic storage, magnetic storage, etc. The storage may comprise local storage, e.g., a local hard drive or electronic memory. Storage 14 and 34 may comprise non-local storage, e.g., cloud storage. In the latter case, storage 14 and 34 may comprise a storage interface to the non-local storage. Storage may comprise multiple discrete sub-storages together making up storage 14, 34. Storage may comprise a volatile writable part, say a RAM, a non-volatile writable part, e.g., Flash, a non-volatile non-writable part, e.g., ROM.

Storage 14 and 34 may be non-transitory storage. For example, storage 14 and 34 may store data in the presence of power such as a volatile memory device, e.g., a Random Access Memory (RAM). For example, storage 14 and 34 may store data in the presence of power as well as outside the presence of power such as a non-volatile memory device, e.g., Flash memory.

Pose estimator training device may have access to a database, which may be used to store training data sets, and the like.

The devices 11 and 31 may communicate internally, with each other, with other devices, external storage, input devices, output devices, and/or one or more sensors over a computer network. The computer network may be an internet, an intranet, a LAN, a WLAN, etc. The computer network may be the Internet. The devices 11 and 31 comprise a connection interface which is arranged to communicate within system 10 or outside of system 10 as needed. For example, the connection interface may comprise a connector, e.g., a wired connector, e.g., an Ethernet connector, an optical connector, etc., or a wireless connector, e.g., an antenna, e.g., a Wi-Fi, 4G or 5G antenna. The communication interface 15 may be used to send or receive digital data, e.g., training data sets, initial pose estimators, further pose estimators, etc. The communication interface 35 may be used to send or receive digital data, e.g., sensor measurements, poses, etc. The communication interface 35 may be used to communicate with one or more sensors and/or one or more controllers.

The execution of devices 11 and 31 may be implemented in a processor system. The devices 11 and 31 may comprise functional units to implement aspects of embodiments. The functional units may be part of the processor system. For example, functional units shown herein may be wholly or partially implemented in computer instructions that are stored in a storage of the device and executable by the processor system.

The processor system may comprise one or more processor circuits, e.g., microprocessors, CPUs, GPUs, etc. Devices 11 and 31 may comprise multiple processors. A processor circuit may be implemented in a distributed fashion, e.g., as multiple sub-processor circuits. For example, devices 11 and 31 may use cloud computing.

Typically, the pose estimator training device 11 and pose estimator device 31 each comprise a microprocessor which executes appropriate software stored at the device; for example, that software may have been downloaded and/or stored in a corresponding memory, e.g., a volatile memory such as RAM or a non-volatile memory such as Flash.

Instead of using software to implement a function, the devices 11 and/or 31 may, in whole or in part, be implemented in programmable logic, e.g., as field-programmable gate array (FPGA). The devices may be implemented, in whole or in part, as a so-called application-specific integrated circuit (ASIC), e.g., an integrated circuit (IC) customized for their particular use. For example, the circuits may be implemented in CMOS, e.g., using a hardware description language such as Verilog, VHDL, etc. In particular, pose estimator training device 11 and pose estimator device 31 may comprise circuits, e.g., for cryptographic processing, and/or arithmetic processing. In hybrid embodiments, functional units are implemented partially in hardware, e.g., as coprocessors, e.g., neural network coprocessors, and partially in software stored and executed on the device.

**Figure 1****.****3** schematically shows an example of an embodiment of a robotic system 40. System 10 may comprise multiple pose estimator training devices; shown are a sensor 41, e.g., a camera, e.g., a visible light camera, e.g., a radar, e.g., a LIDAR; a controller 42 comprising a pose estimator 31; and an actuator 44. Controller 42 receives a sensor measurement from sensor 41 and applies the pose estimator device 31 to it, thus obtaining a pose. The pose is used as a further input to derive a control signal 43. For example, the robotic system may be an autonomous vehicle and the pose may be used to predict a trajectory, e.g., course, for an object, e.g., of a pedestrian, bicycle, vehicle. For example, wheel position, upper body position, etc., may be used to predict a future trajectory. The control signal 43 may be adapted, e.g., for collision avoidance. For example, the robotic system may be arranged to manipulate the object, e.g., in a manufacturing machine, e.g., the robotic system may comprise a robotic arm configured to manipulate the object. The pose of the object may be used to derive how the object needs to be manipulated so that a desired pose is obtained, e.g., so that a next manufacturing step may be performed.

Control signal 43 may be sent from controller 42 to actuator 44, e.g., over a local computer network, a wireless transmission, or the like.

A pose estimator may be used for analyzing data obtained from a sensor. The sensor may determine measurements of the environment in the form of sensor signals, which may be given by, e.g., digital images, e.g., video, radar, LiDAR, ultrasonic, motion, thermal images, or point clouds. Extensive experiments were performed on camera images and human poses. These are discussed herein. However, embodiments apply to other types of sensor measurements, and other types of objects with their corresponding poses. The sensor measurement may comprise a still image or a video sequence. The sensor measurement may comprise a 2D image, a depth image, e.g., obtained from a depth camera or an RGB-D sensor, e.g., a 3D image, a radar measurement, a lidar measurement, e.g., represented as a point cloud, or even pose estimates from a motion-capture system, e.g., based on reflective markers, inertial-measurement units, IMUs, etc. A still image comprised in a sensor measurement may be obtained from a video sequence.

A pose estimator may be used for Controlling a Technical System, for example, to compute a control signal for controlling a technical system, like e.g., a computer-controlled machine, like a robotic system, a vehicle, a domestic appliance, a power tool, a manufacturing machine, a personal assistant. The output of a pose estimator can be used to generate a control signal for such systems via some additional processing.

A pose estimator may be used in a system for conveying information, like a surveillance system, e.g., by providing more fine-granular information than pure human detection. A pose estimator may be used in a medical imaging system. For example, human pose estimation may be used for medical purposes, e.g., to analyze behavior of disabled patients.

A pose estimator may be combined with a control algorithm, especially a known control algorithm configured to use a pose as one of its inputs. As a pose estimator according to an embodiment has a higher accuracy, the control algorithm is also improved. For example, pose may be used as an input for accurate collision avoidance in the vicinity of humans, or human-robot collaboration tasks, e.g., handing over an item to a human, or vice versa. The latter example may use both an estimated human pose as well as an estimated object pose.

A robotic system may compute a 3D trajectory around humans and their body parts estimated by a pose estimator according to an embodiment, and control an autonomous vehicle to follow this trajectory such that the vehicle does not hit any, or any specific, part of a body. This applies to any mobile or stationary robot or vehicle operating in the vicinity of humans.

A pose estimated by a pose estimator, e.g., a set of multiple estimated key points, according to a pose format, can be provided to a downstream processing module, e.g., the control unit of, e.g., an autonomous vehicle, or to a user interface, a database, or to another dependent component in applications related to, for example, autonomous driving, robotics, surveillance, computer graphics, entertainment, medicine, or sports, in order to display information to a user, store information in a database, or send control inputs to some actuator(s) to trigger an action, based on the presence or location of certain key points in the input data.

In an embodiment, training a pose estimator may use three phases. In a first phase, initial pose estimators are obtained that may be specialized for a particular pose format. In a second phase, an autoencoder is trained to transform between pose formats. In this phase, the initial pose estimators are used to obtain poses for the same sensor measurement according to different pose formats-these are then used to train the autoencoder. In a third phase, the trained autoencoder is used to train a further pose estimator which, using the trained autoencoder, can take into account more than one, or even all, training data sets. Below an embodiment of training a pose estimator is described with reference to the figures.

Embodiments use multiple training data sets. Training data sets may be obtained from collection methods like capture setups, synthetic data generation, annotating images, etc. The training data sets comprise sensor measurements, e.g., images, data from inertial measurement units (IMUs), point clouds, etc., and ground truth reference poses, e.g., from motion capture, manual annotation, etc. These reference poses use different pose formats, e.g., use a different number or definition of key points. No prior knowledge of how the pose formats of the multiple training data sets relate to each other is needed or assumed; e.g., how a pose, e.g., skeleton, from one set relates to another.

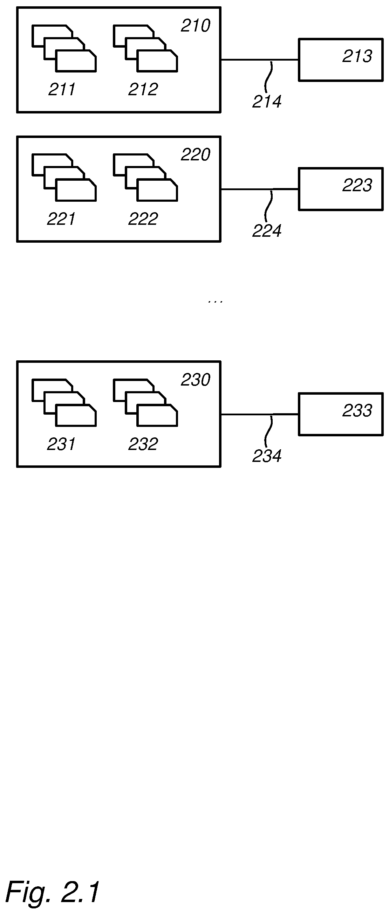

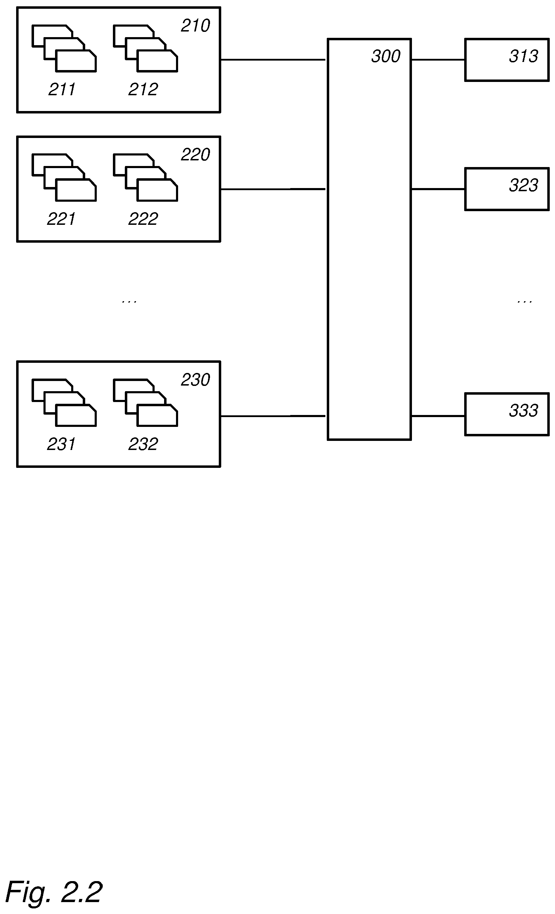

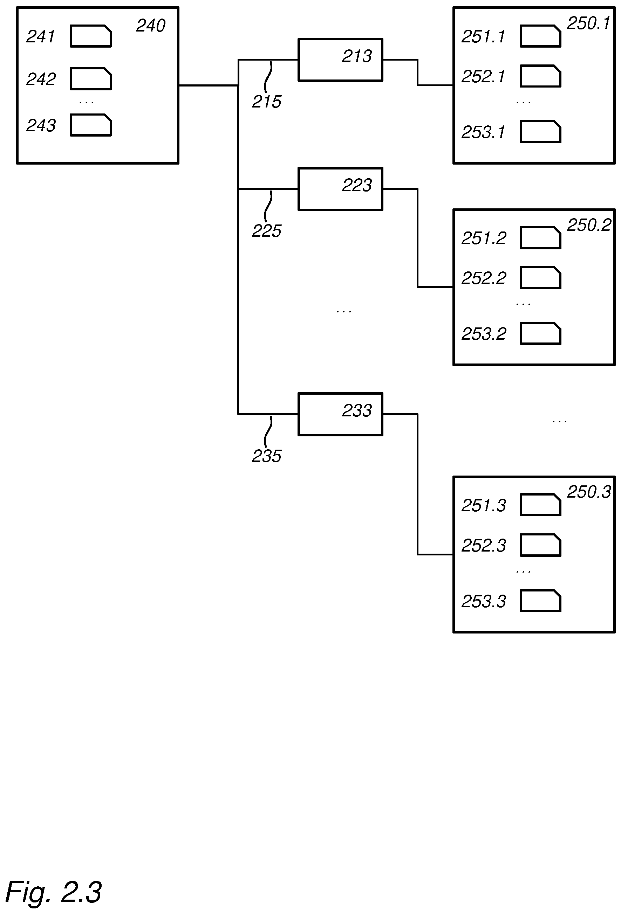

**Figure 2****.****1** schematically shows an example of an embodiment of multiple training data sets and multiple initial pose estimators. Obtaining multiple training data sets may comprise receiving sensor measurements from a sensor and corresponding poses. Typically, however, multiple training data sets are received from storage.

Figure 2.1 shows multiple training data sets. Shown is training data set 210 comprising sensor measurements 211 and corresponding poses 212; training data set 220 comprising sensor measurements 221 and corresponding poses 222; training data set 230 comprising sensor measurements 231 and corresponding poses 232. The pose formats of poses 212, 222, and 232 may be different. For example, there may be more or fewer key points. For example, the order of the key points may be different. Even if two corresponding key points are identified, they may not measure exactly the same landmark, e.g., one key point may be a corner edge of a shoulder, while another may be a center point of the shoulder. The exact definition of a pose format may not be digitally accessible, or may not even be known.

The number of training data sets may be, e.g., at least 10, at least 20, at least 100. The number of training pairs in a training data set may be at least 100, at least 1000, at least 10000, at least 100000.

One may assume that training sets sharing the same pose format are combined. This is convenient but not necessary.

For each of the training data sets, e.g., for each of the training data sets having a different pose format, an initial pose estimator is obtained. The trained initial pose estimator of the multiple trained initial pose estimators may be trained on a corresponding training data set of the multiple training data sets.

Shown in figures 2.1 is: an initial pose estimator 213 trained 214 on training data set 210, an initial pose estimator 223 trained 224 on training data set 220, an initial pose estimator 233 trained 234 on training data set 230.

As the initial pose estimators are trained on their respective training data sets, they produce poses according to the pose conventions, e.g., pose format of the training data set. For example, the initial pose estimators may generate a plurality of key points identified in the object as points in 3D space. For example, a pose may be implemented as a list of estimated key points.

The initial pose estimators are configured to produce an estimated pose for an object represented in a sensor measurement. A pose format can be defined for all kinds of articulated and non-articulated objects, e.g., humans, animals, the corners of a box, vehicles, or a pallet truck with a movable handle. Vehicles include, e.g., cars, trucks, bicycles, and forklifts. Typically, objects represented in the training data sets, and for which the initial pose estimator are trained, are objects of the same type. For example, all training data sets may all comprise sensor measurements showing a human, a car, or the like. However, it is possible that a pose estimator, e.g., the multiple initial pose estimators, are trained to produce a pose estimate for multiple object types.

Typically, the initial pose estimators comprise a neural network. This is not necessary though, especially for the initial pose estimators, any pose estimation methods may be supported, including pre-neural network estimators. Training the initial pose estimators may follow a conventional pose estimator training method. For example, the initial pose estimators may be trained using supervised learning by minimizing a pose loss function, e.g., as is conventionally used to train a pose estimator. The pose loss may comprise, e.g., relative and/or absolute 2D and/or 3D key point losses. For example, a key point loss may be realized as an *ℓ*₁ loss. The pose loss may comprise a projection and/or reprojection losses. Other losses may be included, which may be weighted with weighting factors.

**Figure 2.2** schematically shows an example of an embodiment of multiple training data sets, a shared backbone and multiple prediction heads. The initial pose estimators, e.g., pose estimators 213-233, may comprise an ensemble of independent pose estimators. For example, two independent pose estimators may not share any parameters. An advantageous way to train the initial pose estimators which is shown in figure 2.2 is to use a shared backbone 300 for all initial pose estimators and a prediction head for each specific pose format, e.g., for each training data set. A specific prediction head is only trained on the specific corresponding training data set(s), while the shared backbone 300 may be trained on all training data sets.

Optionally, multiple training data sets may share one prediction head if they use an identical pose format. Such a prediction head may be trained on the corresponding multiple training data sets. It is not necessary in practice that multiple training data sets are merged even if they share the same pose format. For convenience, it is assumed that there is one training data set for each pose format though. Embodiments can easily be adapted should multiple training data sets be available for a given pose format.

Shared backbone 300 may comprise a shared neural network, which may serve as the backbone of the initial pose estimators, and may later be used for the further pose estimator.

Embodiments may be employed for two training data sets, rather than the three shown in figure 2.1, or on more than three training data sets.

Shared backbone 300 is configured to receive the sensor measurement and to produce an output in a latent space. A prediction head of the multiple prediction heads receives as input the latent space output of the shared backbone 300, and produces as output an estimated pose in the pose format of its corresponding training data set. Using a shared backbone 300 already achieves some of the advantages of training with multiple training data sets. As the shared backbone 300 performs an important part in estimating the pose, the prediction for a particular pose format is improved even if the corresponding prediction head is only trained on the subset of the training material available to train the shared backbone 300. However, experiments showed that while the information sharing improved the projected 2D poses, the separate output heads for different pose formats generate inconsistent depth estimates. Figure 2.2 shows a shared backbone 300, and separate prediction heads: a prediction head 313 for training data set 210, a prediction head 323 for training data set 220, and a prediction head 333 for training data set 230.

**Figure 2****.****3** schematically shows an example of an embodiment of a pool of sensor measurements. To train an autoencoder that can transform poses from one format to another, a pool of sensor measurements is selected. Like the training data set, a sensor measurement in the pool represents the object. For example, the object may be a human, a car, bicycle, box, manufacturing item and so on.

Shown in figure 2.3 is a pool 240 of sensor measurements, e.g., a set of sensor measurements. The pool should be large enough to provide a sufficient number of training data for the autoencoder. As the number of parameters in the autoencoder could be much lower than that of a pose estimator, the number of sensor measurements in the pool could also be smaller, e.g., at least 50, at least 100, e.g., at most 1000, at most 10000, sensor measurements.

Figure 2.3 shows a sensor measurement 241, 242 and 243 in pool 240. For example, the sensor measurements 241-243 may be images or any of the other example set out herein.

The multiple trained initial pose estimators are applied to the sensor measurements in the pool. In this way, multiple estimated poses are obtained for each sensor measurement in the pool. Shown in figure 2.3 is applying 215 initial pose estimator 213 on pool 240 to obtain set 250.1 of estimated poses. Set 250.1 of estimated poses contains a pose for each sensor measurement in pool 240. Shown are pose 251.1, pose 252.1, and pose 253.1, corresponding to sensor measurements 241, 242 and 243. Similarly, pose estimators 223 and 233 are applied to the sensor measurements in pool 240. Note that the pose estimators may have a shared backbone and a specific prediction head.

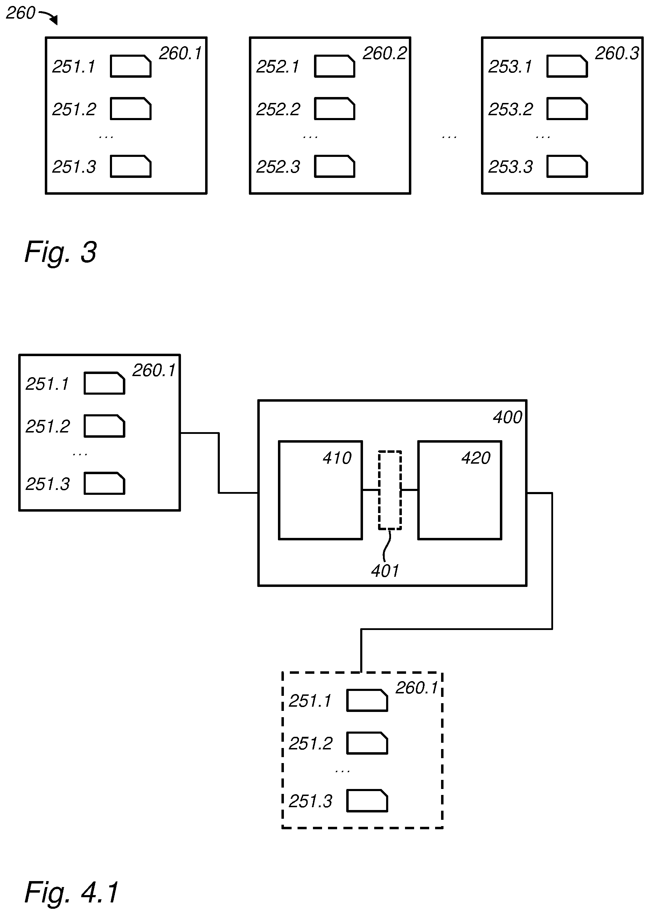

**Figure 3** schematically shows an example of an embodiment of autoencoder training data 260. An autoencoder training data element comprises a set of estimated poses that correspond to the same sensor measurement. The estimated poses in the same autoencoder training data element were obtained from different initial pose estimators. For example, autoencoder training data element 260.1 comprises poses 251.1, 251.2, 251.3 corresponding to the same sensor measurement 241. The poses in the autoencoder correspond to the poses used by the training data set, and initial pose estimators.

Likewise training data set element 260.2 gives a pose for the object in sensor measurement 242, but in different pose formats. Likewise for training data set element 260.3. There are as many autoencoder training data as there are sensor measurements in pool 240.

Accordingly, the initial pose estimators may be used to generate new training data, sometimes referred to as pseudo-ground truth data. In this way multiple poses in multiple pose formats are obtained for the same sensor measurement. The input training data sets used for this pseudo-ground truth generation step may be selected by hand or automatically, e.g., via appropriate metrics.

The sensor measurements in pool 240 could be selected randomly, e.g., randomly from any of the multiple training data sets, or from their union. However, better results are obtained by making a more careful selection of the sensor measurements in pool 240. Preferably, the sensor measurements in pool 240 are of high quality so that a pose estimator is expected to give an accurate pose estimate for it. For example, a sensor measurement in pool 240 is preferably free of clutter, occlusion and key point location noise. There are various ways to improve the selection of pool 240.

Selection of sensor measurements for the pool are conveniently made from the one or more of the training data sets, or from the union of the training data sets. It is not necessary that all training data sets are represented. It is also not needed that the same sensor measurements were used in training either.

In an embodiment, a training data set comprises a sequence of sensor measurements. For example, images sensor measurements may be obtained from a video, e.g., of a moving subject, e.g., to obtain human poses. For example, sensor measurements may be recorded while driving a vehicle, e.g., images or lidar measurements may be recorded while driving a vehicle, etc. Some of the sensor measurements may be of good quality and some of lesser quality. This can be detected by applying a trained initial pose estimator and determining a deviation between poses in the training data set for sensor measurements in the sequence. If the deviation is high, then it is likely that something was wrong, probably an occlusion. While if poses of subsequent sensor measurements are in agreement, then there is a good chance these sensor measurements are favorable. In an embodiment, a deviation is computed, e.g., a standard deviation for the poses, e.g., their underlying key points, and sensor measurements are selected for the pool that have a deviation below a threshold. For example, a designer of the system may select the threshold. For example, the threshold may be selected so that only the best n sensor measurements are selected, where n is say 100, 1000, etc.

In an embodiment, a motion model may be applied to predict poses one or multiple time steps into the past or future. If the motion model is confident of the prediction, and/or, predicted poses match observed poses well, then this is an indication that the poses in this part of the sequence are good examples.

In an embodiment, selecting for the pool comprises determining an occlusion of the object. Occlusions are particularly problematic for training the autoencoder as they introduce noise into the computed poses, e.g., poses 251.1-253.3. Occlusion can be detected, e.g., by training an image classifier on recognizing occlusions, or through geometric 3D reasoning by performing ray-casting and computing intersections with other known, sensed or detected foreground objects, e.g., using a point cloud-, voxel-, mesh- or Octree-based representation and techniques known from computer graphics. This can be done with high accuracy. Sensor measurement may be selected for the pool that have occlusion below a threshold, e.g., the best n may be selected.

In an embodiment, an initial pose estimator may be configured to provide an accuracy value, e.g., a confidence value, as well as an estimated pose. The accuracy value indicates the likelihood that the pose is correct. Accuracy values may be obtained from other sources, e.g., operator evaluation or the like. Accuracy values may also be provided individually for each key point and then averaged to obtain an overall accuracy for a pose. An embodiment may comprise selecting for the pool a sensor measurement having an accuracy above a threshold.

These indicators of quality may be combined, for example, sensor measurements obtained with low occlusion and high accuracy, e.g., confidence, etc.

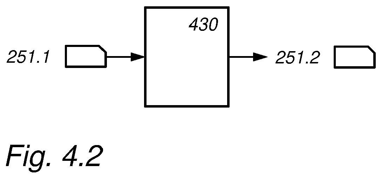

An autoencoder may be trained on the multiple estimated poses, e.g., on training data 260. The autoencoder may be trained to receive at least a first pose according to a first pose format and to produce at least one second pose according to a second pose format. For example, the first pose format be that of a first training data set, while the second pose format be that of a second training data set. Having the ability to transform poses from one pose format to another allows a further pose estimator to be trained on multiple training data sets, since, if a pose is not in the format expected by the pose estimator, then the autoencoder can transform it to another pose format, which is expected. **Figure 4****.****2** schematically shows an example of an embodiment of such a minimal autoencoder 430. Shown in figure 4.2 is an autoencoder 430 receiving as input a pose in a first format, in this example pose 251.1, and as output a pose in a second format, in this example pose 251.2. Possibly, multiple autoencoders may be trained if transformation in different directions are required.

**Figure 4****.****1** schematically shows an example of an embodiment of an autoencoder 400 that is configured to receive as input multiple poses for the same sensor measurement according to the pose format of multiple training data sets; shown in figure 4.1 is autoencoder training data element 260.1. For example, the autoencoder may comprise
- an encoder 410 configured to receive as input the multiple poses, and to produce a representation in a latent space 401, and
- a decoder 420 configured to receive as input the representation in the latent space 401, and to produce the multiple poses as output.

The output of autoencoder 400 should be close to the input of the autoencoder. As they are typically not fully identical, the output of autoencoder 400 has been indicated with a dashed line.

In an embodiment, encoder 410, decoder 420, or both encoder and decoder, are linear transformations. What is more, encoder 410, decoder 420, or both encoder and decoder may be restricted to compute combination of points. For example, encoder 410 may be configured to receive a sequence of key points, e.g., typically 3D points, and to generate a sequence of points in the latent space. Generating a point in the latent space may be restricted to a linear combination of input points. Likewise, decoder 420 may be configured to receive a sequence of latent points, e.g., typically 3D points, and to generate a sequence of output points in the latent space. Generating a point in the latent space may be restricted to a linear combination of input points. The autoencoder may be implemented such that it takes as input one or multiple lists of estimated key points from the pose estimator according to one or multiple pose formats and encodes them into latent key points by computing combinations q*ₗ* of all of these input key points using, e.g., the following equation: ${q}_{l} = {\sum }_{j = 1}^{J} {w}_{l , j}^{enc} {p}_{j} , preferably such that {\sum }_{j = 1}^{J} {w}_{l , j}^{enc} = 1 , ∀ l = 1 , … , L .$ where *J* is the number of input key points p*ⱼ*, L is a hyperparameter determining the number of latent key points, and ${w}_{l , j}^{enc}$ are the learnable parameters of the encoder part, which represent combination weights. These learnable weights are also representable as *W*_{enc} in matrix form.

Similarly, the decoder reconstructs key points p̂*ⱼ* corresponding to the original pose formats from these latent key points via a combination of the latent key points using, e.g., the following equation: ${\hat{p}}_{j} = {\sum }_{l = 1}^{L} {w}_{j , l}^{dec} {q}_{l} , preferably such that {\sum }_{l = 1}^{L} {w}_{j , l}^{dec} = 1 , ∀ j = 1 , … , J .$ where q*ₗ* are the latent key points encoded in the previous equation, and ${w}_{l , j}^{dec}$ are the learnable parameters of the decoder part, which represent combination weights. Again these learnable weights are also representable as *W*_{dec} in matrix form. The decoder thus outputs the reconstructed key points as one or multiple lists corresponding to the original pose formats.

In an embodiment the autoencoder takes as input a pose according to each of the pose formats, and produces as output a pose according to each of the pose formats. But this is not necessary, the above equations can be used to transform between any two pose formats, or between any two sets of pose formats, as desired.

Accordingly, in an embodiment, the latent space can be represented as a sequence of points in 3D space. This has the advantage that latent points can be shown in the same space as estimated poses are. The number of points in the latent space *L* is a hyperparameter, which may be selected empirically. For human poses, a body has about 30 joints, so about 30 key points are expected for a pose format, or possibly a bit over, or a bit under. For example, for a human pose estimator the number of latent points may be at least 30, or at least 40. For example, for a human pose estimator the dimension of the latent representation of a set of poses may be at least 90. Note that the input size and output size may be a lot larger. For example, assuming typical numbers, e.g., about 30 key points in a human pose, and 20 training data sets with distinct pose formats, the input will have 600 points, or a dimension of 1800; the output space may be the same.

Preferably, the encoding and decoding weights each sum to 1, that is, they represent an affine combination. Affine combination provides translation equivariance, which is desirable for a pose transformation. A pose transformation preferably does not change if the pose is moved in 3D space.

In an alternative implementation, for both the encoder and decoder part, there could also be nonlinear operations involved instead of considering only affine combinations. For example, the autoencoder may comprise a neural network. For example, the encoder and/or the decoder may comprise a multiple layer neural network.

Some objects for which a pose is computed have a chirality equivalence; as an example, consider an embodiment in which the key points of each pose format can be portioned into three parts: Left, Middle, Right, e.g., for a human pose estimator. The pose transformation may then be expected to use the same parameters for Left points, as for Right points. Such conditions may be imposed on the parameters of the autoencoder, especially upon a linear autoencoder such as the affine, combining autoencoder. In an embodiment, training the autoencoder is subject to one or more equality conditions on parameters of the autoencoder ensuring chirality equivariance. For example, the left and right hand of a human are an example of two sets that are expected to be transformed similarly.

Chirality constraints may be added to the weight matrices *W*_{enc} and *W*_{dec}. For example, for bilateral symmetry as in the example of human key points, the set of latent of key points may be divided into three disjoint sets (left, central, right). Then the key points may, without loss of generality, be sorted and grouped into left-side, right-side and center matrix blocks. Then, a weight-sharing matrix block structure, for instance represented as follows, may be imposed on the weight matrices *W*_{enc} and *W*_{dec} to ensure chirality equivariance: $W = \left[\begin{matrix}{W}_{1} & {W}_{2} & {W}_{3} \\ {W}_{2} & {W}_{1} & {W}_{3} \\ {W}_{4} & {W}_{4} & {W}_{5}\end{matrix}\right]$ where *Wₓ* represent sub-matrices, e.g., blocks of the full weight matrix *W*_{enc} or *W*_{dec}.

In an embodiment, the autoencoder, whether linear or not, may comprise minimizing a combination of one or more of a reconstruction loss, a sparsity loss, a reconstruction loss in a projected space, and/or further losses, possibly be weighted via weighting factors. For example, the Adam optimization algorithm may be used to minimize the resulting loss function.

The sparsity loss encourages sparse weights in the encoder and decoder part of the autoencoder and may, for instance, be implemented via ℓ₁ regularization: ${L}_{sparse} = {\left‖{W}_{enc}\right‖}_{1} + {\left‖{W}_{dec}\right‖}_{1}$

The reconstruction loss for the autoencoder reconstructs close-to-original key point estimates from its input key point estimates and may, for instance, be implemented via ℓ₁ regularization: ${L}_{reconstr} = \frac{1}{K} {\sum }_{k = 1}^{K} {\left‖{P}_{k}-{W}_{dec}{W}_{enc}{P}_{k}\right‖}_{1}$ where *K* is the total number of input training samples, e.g., training poses. Each training sample consists of *J* key points, which usually covers multiple pose formats, and may be represented in matrix form as ${P}_{k} ∈ {ℝ}^{J \times D}$*,* where *D* is the dimensionality of a key point, e.g., 3 for a 3D point.

In a variant, particularly when *D* > 2, the key points may first be projected into a lower-dimensional space prior to computing the reconstruction loss using an alternative version of the above formula, for example: ${L}_{reconstr}^{proj} = \frac{1}{K} {\sum }_{k = 1}^{K} {\left‖\prod \left({P}_{k}\right)-\prod \left({W}_{dec}{W}_{enc}{P}_{k}\right)\right‖}_{1}$ where Π is a function that projects the key point coordinates into a lower-dimensional space, for example 2D image space.

Having a trained autoencoder a further pose estimator may be trained. At this point the learned weights of the autoencoder may be frozen. A new further pose estimator may be trained from scratch, or an existing pose estimator may be fine-tuned. In particular, an architecture as shown in figure 2.2 may be fine-tuned using the autoencoder. Typically, the shared backbone and multiple heads each comprise layers of a neural network. These may be fine-tuned using the autoencoder. The further pose estimator provides more accurate and consistent key point estimates.

Using the autoencoder allows incorporation of geometric constraints and/or regularization losses into the training of the pose estimator such that the resulting key points predictions eventually respect the relations and redundancies discovered by the affine-combining autoencoder in the previous training step.

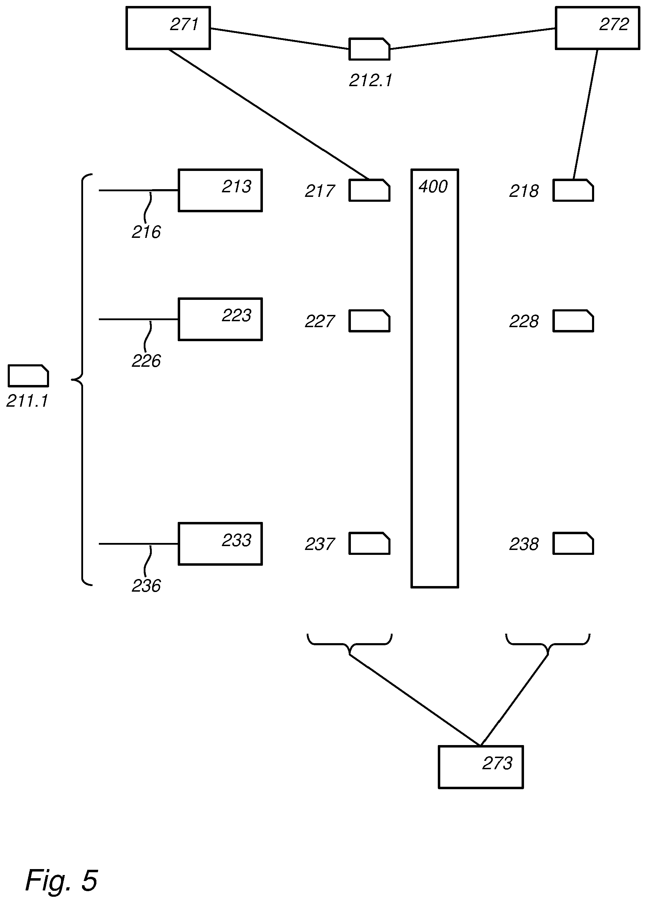

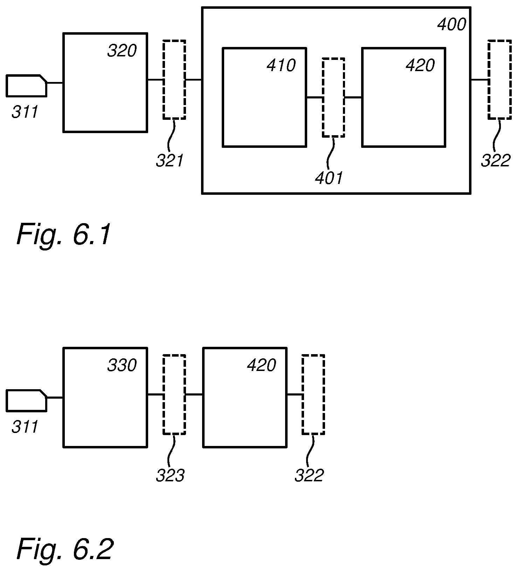

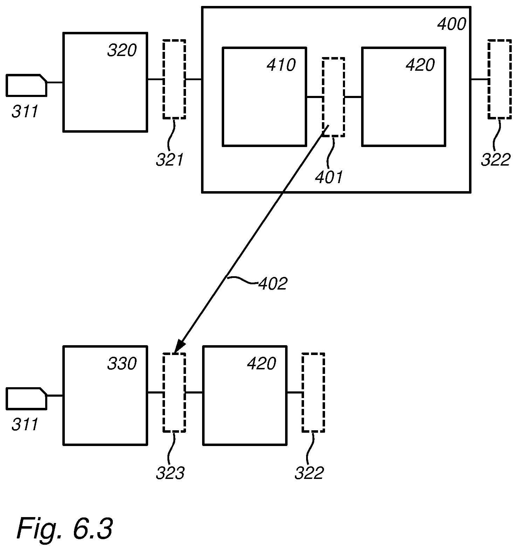

**Figure 5** schematically shows an example of an embodiment of pose estimators. **Figure 6****.****1** schematically shows an example of an embodiment of pose estimators. Figure 5 and 6.1 show a similar embodiment, highlighting different features. Figures 6.2 and 6.3 show different variants of training a further pose estimator.

By training the further pose estimator on more than one of the multiple training data sets, using at least part of the trained autoencoder, the pose estimation is improved. While using a shared backbone with multiple prediction heads gives some transfer of knowledge across training sets, the effect is limited, and can be improved by having explicit losses relating the poses generated for different training sets.

It is possible to use a full autoencoder for this, e.g., an autoencoder receiving a pose for each of the pose formats, and producing a pose for each of the pose formats. For example, the further pose estimator may be configured to receive as input a sensor measurement and to produce as output a pose according to the multiple pose formats corresponding to the multiple training data sets. The full autoencoder may be used to train the further pose estimators, by providing additional losses for optimization, e.g., a consistency loss, and an additional pose loss, as described herein. The consistency loss provides regularization to the pose estimator based on the latent structure learned by the autoencoder.

It is not needed to use a full autoencoder for training. For example, even a limited autoencoder that transfers from a pose format, e.g., one or few, to another pose format, e.g., one or few, is helpful in further training. The limited autoencoder may be used to transform training data from one pose format to another, so that the training data can be used for a different pose format.

It is also not needed to use the whole autoencoder for training. For example, one could use only the decoder from an autoencoder as in figure 4.1.

During training of the further pose estimator, whether from scratch or fine-tuning, the autoencoder is typically frozen. Is possible to iterate training of pose estimators with training of the autoencoder. For example, once improved further pose estimators are available for multiple pose formats, new training data as in figure 2.3 and/or figure 3 may be generated, and a further autoencoder may be trained. The further autoencoder can then be used for further training, e.g., fine-tuning, of the pose estimators. Such iterations are however not necessary, and good results have been obtained with a single autoencoder.

Figure 6.1 shows a pose estimator 320 having a shared backbone and multiple prediction heads. Given an input sensor measurement 311, each prediction head produces a pose according to its pose format. Estimated poses 321 comprises multiple poses, e.g., as a multiple poses vector 320. Typically, multiple poses vector 320 comprises one pose for each of the pose formats available in the training data sets. Estimated poses vector 321 is input to autoencoder 400. In this case, an encoder/decoder autoencoder is used, as shown in figure 4.1. The output 322 of autoencoder 400 preferably is close to estimated poses 321. Typically, output 322 is also a multiple poses vector comprising one pose for each of the pose formats available in the training data sets. The output of the autoencoder is also referred to as the reconstructed poses, as they are reconstructed from a latent representation. Note that this is not necessary, in an embodiment either one of multiple poses vectors 321 and 322 could comprise fewer than all available pose formats. Pose estimator 320 could be trained from scratch or could be fine-tuned, e.g., from the initial pose estimators.

Figure 5 shows a similar situation. Shown is a sensor measurement, as an example, sensor measurement 211.1 is taken. The initial pose estimators 213-233 are applied to the sensor measurement 211.1. The initial pose estimators are fine-tuned in this example. The initial pose estimators 213-233 may be as in figure 2.2; having a shared backbone and multiple prediction heads. For example, pose estimator 320 may be the collection of pose estimators 213-233.

The multiple initial pose estimators generate estimated poses 217, 227, 237. The collection of estimated poses is used as input to the autoencoder 400. This produces a new set of estimated poses 218, 228, 238. If the multiple initial pose estimators were accurate and consistent with each other, then the new set of estimated poses 218, 228, 238 would be expected to be close to the original set of generated estimated poses 217, 227, 237.

The multiple initial pose estimators may be trained, e.g., fine-tuned, on various losses. As before the initial pose estimators may be trained on pose losses. For example, as 211.1 comes from training data set 210, a corresponding pose 212.2 is known for it. Accordingly, a loss term 271 may represent the distance between estimated pose 217 and known pose 212.2. In this case an additional pose loss 272 is available, namely based on the distance between known pose 212.2 and reconstructed pose estimate 218 after processing through the autoencoder. By selecting the sensor measurement from the various training sets, known poses are obtained from the various training set, and thus a pose loss training signal is available for each of the pose estimators 213-233. The different types of pose losses described herein, are applicable in this setting. In addition to pose losses, also a consistency loss 273 may be used. That is, the set of generated pose estimates 217-237 are preferably close to the set of reconstructed pose estimates 218-238 after application of the autoencoder.

Together, the additional pose and consistency losses may provide an autoencoder-based regularization during training of the further pose estimator 320 to provide additional supervision and ensure consistency of pose formats.

In the context of figure 6.1, pose estimator 320 may be trained with pose losses based on the part of multiple poses vector 320 and/or 322 that corresponds to the known pose. Pose estimator 320 may be trained with a consistency loss based on the distance between multiple poses vector 320 and 322.

A consistency loss, e.g., between vectors 321 and 322, or pose estimates 217-237 and reconstructed pose estimates 218-238, encourages consistency between the directly predicted key points from the further pose estimator and the reconstructed key points of the autoencoder. A consistency loss may be implemented via *ℓ*₁ regularization: ${L}_{cons} = {\left‖\hat{P}-{W}_{dec}{W}_{enc}\hat{P}\right‖}_{1}$ where *P̂* is the model prediction of the further pose estimator, e.g., in matrix form, e.g., vector 321, and the second term *W_{dec}W_{enc}P̂* yields the reconstructed key point estimates of the autoencoder, e.g., in matrix form.

As described before, pose losses, e.g., 271 and 272, may be applied on both the model prediction *P̂*, e.g., and the reconstruction *W_{dec}W_{enc}P̂* of the autoencoder.

In the later inference step, e.g., in an operational use of the final trained pose estimator according to figure 6.1, the model prediction *P̂*, e.g., vector 321, may, e.g., be used as the final output, and the autoencoder may be removed from the network. If the pose estimator predicts poses in multiple formats, e.g., by having multiple prediction heads, then heads that predict poses in unneeded pose formats may also be removed.

Typically, all heads, but one are removed, as well as the autoencoder, in the final pose estimator of this embodiment.

In an embodiment, dimensionality reduction of the key points in multiple poses uses an affine-combining autoencoder. The multiple poses are represented as a sequence of points in a latent 3D space. By regularizing a pose estimator's output during training or fine-tuning, e.g., via an additional pose and consistency loss, information can be shared efficiently between different training data sets, resulting in an overall more accurate and consistent pose estimator. The discovered latent 3D points capture the redundancy among pose formats, enabling enhanced information sharing when used for consistency regularization during training of the further pose estimator 320. For example, multiple pose estimates may be encoded into the latent space via the frozen encoder, which may be a subspace of the full pose space. The multiple pose estimates are then reconstructed by the frozen decoder. The reconstructed poses may not exactly match the input pose estimates because of the constraints imposed by the latent representation. During fine-tuning, the consistency loss, optionally together with the 2^{nd} pose loss at the auto-encoder output, feeds back or backpropagates these constraints learned by the autoencoder to the pose estimator. In other words, the additional loss terms help to backpropagate the latent structure learned by the autoencoder to the further pose estimator during its training, acting as additional supervision signals.

**Figure 6****.****2** schematically shows an example of an embodiment of pose estimators. Shown in figure 6.2 is a pose estimator 330 configured to receive a sensor measurement, e.g., like, pose estimator 320. However, instead of directly generating a pose, or even multiple poses, like pose estimator 320, pose estimator 330 predicts the pose representation, e.g., vector 323, in the latent space of the autoencoder. The decoder part 420 of the autoencoder may then be applied to the latent representation, e.g., to produce a multiple poses vector 322. The decoder could also be restricted to generate only one of the multiple poses. The further pose estimator 330 can now be trained on each one of the training data sets, e.g., using a pose loss.

For example, training pose estimator 330 may comprise minimizing one or more losses, for example, one or more pose losses. A pose loss may be obtained from comparing a training pose in the multiple training data sets corresponding to a training sensor measurement to a corresponding output pose of the decoder part of the autoencoder applied to the latent representation of pose estimator 330.

A direct latent prediction pose estimator may be obtained from a shared backbone and heads architecture, as in figure 2.2, by discarding the multiple heads, e.g., head 313-333, and adding a latent prediction head. The combination of shared backbone and latent prediction head may then be trained to predict only a set of L latent key points *Q̂* instead of predicting the full set of *J* key points *P̂* for all pose formats. As the total number of predicted key points in the final neural network layer containing the prediction heads thereby changes from *J* to L, this layer may be initialized and re-learned from scratch. Subsequently, only the decoder part of the autoencoder from the second training phase is used to reconstruct key point estimates in all to be considered pose formats, by attaching the learned decoder with its weights frozen to the new latent prediction head. During the training, a pose loss may be applied to the decoded poses *W*_{dec}*Q̂*. At inference time, the frozen decoder stays attached to the pose estimator, such that the decoded latent key point estimates *W*_{dec}*Q̂* can be used as the final output. The frozen decoder in the final pose estimator may also be restricted to only the desired pose formats, e.g., a single pose format.

Typically, pose estimators 320 and 330 comprise a neural network.

**Figure 6****.****3** schematically shows an example of an embodiment of pose estimators. In figure 6.3, a first further pose estimator 330 is configured to produce an output in a latent space of the autoencoder. This first further pose estimator 330 is similar to the pose estimator of figure 6.2; the same loss terms may be applied here to optimize pose estimator 330. Figure 6.3 also shows a second further pose estimator configured to produce multiple pose formats. The second further pose estimator is similar to the pose estimator of figure 6.1.

In practice it turned out that training a pose estimator that generates a full set of poses, e.g., as in figure 6.1 or figure 5, is easier to train than a pose estimator that only produces a latent representation as in figure 6.2. In this variant, the second pose estimator is used to improve training of the first pose estimator. This may be done by introducing a teacher loss 402. The teacher loss indicates a distance between the latent state 323 predicted by first estimator 330 and the latent representation 401 inside the autoencoder, when pose estimators 320 and 330 are applied to the same sensor measurement 311. For training the second pose estimator, such that it can act as a teacher, the same losses may be used as previously in figure 6.1. The teacher loss 402 is only used to train the first pose estimator, here the student, not the second pose estimator. Pose estimators 320 and 330 could share a shared backbone. For example, the shared backbone may support multiple prediction heads for pose estimator 320, and a prediction head for pose estimator 330. The latter mapping the output of the shared backbone to the latent space of the autoencoder.

The decoder 420 attached to the first pose estimator 330 is the same as the decoder 420 used in the autoencoder 400 attached to the second pose estimator. Typically, the autoencoder 400 including the decoder 420 is frozen during this phase of the training. For example in a shared backbone architecture as in figure 2.2, this variant of training may be achieved by retaining the full set of prediction heads as in figure 6.1 to obtain a multiple estimated poses vector 321, but adding an additional, newly initialized prediction head to predict latent key points *Q̂*, i.e. vector 323, as in figure 6.2. Here, the autoencoder 400 from figure 6.1 acts as a teacher providing supervision to the latent prediction head of pose estimator 330. This can, for example, be achieved by adding a teacher loss that distills knowledge of the full prediction heads to the latent key point prediction head; concretely, the teacher loss term may be directly applied to the two latent key point representations *W_{enc}P̂* and *Q̂*, and be backpropagated only to the student by stopping gradient flow to the teacher. It may, for instance, be implemented via *ℓ*₁ regularization: ${L}_{teach} = {\left‖\hat{Q}-stop_gradient\left({W}_{enc}\hat{P}\right)\right‖}_{1}$

At inference time, the full set of prediction heads of pose estimator 320 and the attached autoencoder 400 acting as a teacher can be discarded. As in the case of figure 6.2, the frozen decoder 420 stays attached to the latent prediction head of the pose estimator 330, such that the decoded latent representation *W*_{dec}*Q̂* can be used as the final output poses.

If at some point an additional training data set becomes available it can be used for additional training. For example, an additional initial pose estimator may be trained, e.g., an additional prediction head may be added to a shared backbone. An autoencoder can now be trained for the further pose format, and training a yet further pose estimator on the further training data set. The new autoencoder could be fully trained again, but instead one could train only an additional part of the encoder and decoder to encode the pose format of the additional pose estimator into and from the existing latent representation.

Below an additional detailed embodiment is provided. Suppose we have *D* pose formats, with ${\left\{{J}_{d}\right\}}_{d = 1}^{D}$ joints in each, for a total of $J = {\sum }_{d = 1}^{D} {J}_{d}$ joints overall. Further, we have a merged dataset with *N* training examples, each example comprising a sensor measurement, in this case an image of a person, and a pose, e.g., annotations for a subset of the *J* body joints in 3D. A first phase comprises training an initial pose estimator to predict all *J* joints separately. This may use a multi-task architectures having different task-specific heads on one backbone. The pose loss we minimize in this example may be 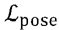 = 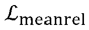 + *λ*ₚᵣₒⱼ 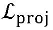 + *λ*_{abs} 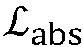 , where 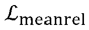 may be an ℓ₁ loss computed after aligning the prediction and ground truth at the mean, 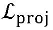 may be an ℓ₁ loss on the 2D coordinates after projection onto the image, and 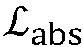 may be an ℓ₁ loss on the absolute pose, e.g., in camera coordinates. A training example may be annotated only with a subset of the *J* joints, in this case any unlabeled joints may be ignored in the loss, e.g., may be ignored when averaging.

The training mechanism used for these initial pose estimators does not use a mechanism to ensure relations between output poses, except that they are predicted from shared backbone features. Indeed, the outputs of the trained models show inconsistencies. In particular inconsistencies are seen along the challenging depth axis. Pseudo-ground truth is generated using the initial separate-head model. It is preferred to use images that the models can handle well in this phase. In an embodiment, images were selected that are a relatively clean, clutter-free subset of the training data. This yields a set of *K* pseudo-ground truth poses, with all *J* joints: ${\left\{{P}_{k}∈{ℝ}^{J \times 3}\right\}}_{k = 1}^{K}$*.*

It is advantageous to select a transformation to and from the latent representation that is viewpoint-independent, since the pseudo-ground truth is more reliable in 2D, e.g., the X and Y axes, than in the depth dimension. For example, an advantageous representation may be equivariant to rotation and translation. This has the advantage that pose formats only depend on how key points are defined, e.g., joints on the human body, not on the camera angle.

For example, a latent representation may be geometric; e.g., a latent representation may comprise a list of *L* latent 3D points ${Q}_{k} ∈ {ℝ}^{L \times 3} \left(L<J\right)$*.* The latent points span the overall structure of a pose. Specific pose formats can then be computed in relation to these latent points.

In an embodiment, sparsity is enforced so that latent points have a sparse influence on key points. A latent point represents a small number of key points; for example, one or few latent points are responsible for the positioning of the left arm and have no influence on the right leg's pose.

An embodiment uses a constrained undercomplete linear autoencoder structure, in particular an autoencoder that uses affine-combining. We refer to this autoencoder as ACAE, which is short for affine-combining autoencoder. The ACAE may comprise an encoder and a decoder. The encoder may take as input a list of *J* points ${p}_{j} ∈ {ℝ}^{3}$ and encode them into *L* latent points ${q}_{l} ∈ {ℝ}^{3}$ by computing affine combinations according to ${q}_{l} = {\sum }_{j = 1}^{J} {w}_{l , j}^{enc} {p}_{j} , {\sum }_{j = 1}^{J} {w}_{l , j}^{enc} = 1 , ∀ l = 1 , … , L .$ (1)

Similarly, the decoder may reconstruct the original points from the latent points, again through affine combinations: ${\hat{p}}_{j} = {\sum }_{l = 1}^{L} {w}_{j , l}^{dec} {q}_{l} , {\sum }_{l = 1}^{L} {w}_{j , l}^{dec} = 1 , ∀ j = 1 , … , J .$ (2)

Since affine combinations are equivariant to any affine transformation, this embodiment is guaranteed to be rotation and translation equivariant. Note that the same weighting may be used for the X, Y and Z coordinates.

The learnable parameters of this ACAE are the affine combination weights ${w}_{l , j}^{enc}$ and ${w}_{j , l}^{dec}$. Negative coordinates may be allowed, as this allows the latent points to spread outwards from the body. Restricting the encoder and decoder to convex combinations is possible but limits its expressiveness. To achieve sparsity in the weights, e.g., a spatially localized influence, ℓ₁ regularization may be used, which also reduces the number of negative weights, as it prefers convex or nearly convex combinations. We further adopt the ℓ₁ reconstruction loss in this embodiment, as it is robust to outliers which may be present due to noise in the pseudo-ground truth.

The ACAE problem for the weights may be stated in matrix notation as follows. Given *K* training poses with *J* joints ${\left\{{P}_{k}∈{ℝ}^{J \times 3}\right\}}_{k = 1}^{K}$*,* $\begin{matrix}{minimize}_{{W}_{enc} ∈ {ℝ}^{L \times J} , {W}_{dec} ∈ {ℝ}^{J \times L}} & {L}_{reconstr} + {λ}_{sparse} {L}_{sparse} \\ {L}_{reconstr} & = \frac{1}{K} {\sum }_{k = 1}^{K} {\left‖{P}_{k}-{W}_{dec}{W}_{enc}{P}_{k}\right‖}_{1} \\ {L}_{sparse} & = {\left‖{W}_{enc}\right‖}_{1} + {\left‖{W}_{dec}\right‖}_{1} \\ s . t . {W}_{enc} {1}_{J} & = {1}_{L} , {W}_{dec} {1}_{L} = {1}_{J} ,\end{matrix}$ (3) where 1*_{α}* is a vector of dimension *a* filled with ones and *λ*ₛₚₐᵣₛₑ controls the strength of the sparsity regularization. The sum-to-one, partitioning of unity, constraints ensure that the weights express affine combinations, this has the advantage of translation equivariance. This condition is preferable, but can be omitted or relaxed.

The pseudo-ground truth is more reliable in its 2D projection than along the depth axis. The above general problem formulation may be adapted to take this into account by defining the reconstruction loss on 2D projections: ${L}_{reconstr}^{proj} = \frac{1}{K} {\sum }_{k = 1}^{K} {\left‖\prod \left({P}_{k}\right) - \prod \left({W}_{dec}{W}_{enc}{P}_{k}\right)\right‖}_{1} ,$ (4) where Π(·) denotes camera projection.

It was found that it is sufficient to observe the high-quality 2D image-plane projections of this model's outputs to characterize how the joints of different pose formats geometrically interrelate, because these relations are viewpoint-independent. As humans have bilateral symmetry, it is natural to expect the autoencoder to be chirality-equivariant, to process the left and right sides the same way. To this end, we partition the latent key points into three disjoint sets: left, right and central latent points, following the same proportions as in the full joint set. Assume, without loss of generality, that the points are sorted and grouped into left-side, right-side and center blocks. We then impose the following weight-sharing block structure on both the encoder and decoder weight matrices: $W = \left[\begin{matrix}{W}_{1} & {W}_{2} & {W}_{3} \\ {W}_{2} & {W}_{1} & {W}_{3} \\ {W}_{4} & {W}_{4} & {W}_{5}\end{matrix}\right] .$ (5)

This structure indeed ensures chirality equivariance, since the matrix remains the same if we permute both its rows and columns by swapping the first two sections, e.g., swapping the left and right points in the inputs and the outputs.

In an embodiment, head and facial key points are weighted in the loss, e.g., by a factor of at least 10. This ensures that the latent point will cover the head well. This feature provides a modest improvement in accuracy for head motions. Weighting of relevant key points may be used for other key points that are important for an application. For example, leg key point and body posture are important for predicting a human trajectory, e.g., course; For example, wheel key point are important for predicting a car's trajectory. In both cases the corresponding key points can be weighted in the loss.

In an embodiment, the autoencoder was trained using Adam optimizer. Once the autoencoder is trained on pseudo-ground truth, its weights may be frozen.

In a subsequent step, the autoencoder may be used to enhance the consistency of 3D pose estimation outputs, by fine-tuning or partly re-training the underlying pose estimator while applying regularization provided by the autoencoder. This can be done in various ways, three of which are presented below.

*Output Regularization:* In this case, we estimate all *J* joints $\hat{P} ∈ {ℝ}^{J \times 3}$ with the underlying pose estimator, but we feed its output through the autoencoder, and during fine-tuning of the pose estimator apply regularization through an additional loss term that measures the consistency of the prediction with the latent space, through an ℓ₁ loss, as ${L}_{cons} = {\left‖\hat{P}-{W}_{dec}{W}_{enc}\hat{P}\right‖}_{1} .$ (6)

This encourages that the separately predicted poses can be projected to latent key points and back without information loss, thereby discouraging inconsistencies between them. The pose loss 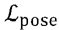 may be applied on *P̂* only, or on *W*_{dec}*W*_{enc}*P̂*, or both. Preferably, the pose loss 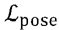 is applied on both. At inference time, either *P̂* or *W*_{dec}*W*_{enc}*P̂* may be used as the final output poses. It is preferable to use *P̂* as the final output poses, since then the computational effort is slightly reduced as the autoencoder can be omitted after it has provided regularization to the pose estimator during fine-tuning.

*Direct Latent Prediction:* To avoid having to predict a large number of *J* joints in the base pose estimator, in an alternative approach the latent points $\hat{Q} ∈ {ℝ}^{L \times 3}$ are directly predicted and then fed to the frozen decoder of the previously trained autoencoder. To realize this, the last layer of the pose estimator may be reinitialized from scratch, as the number of predicted joints changes from *J* to *L.* The pose loss 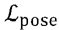 may be applied on *W*_{dec}*Q̂*, which are also used as the final output at inference time. Thus, the frozen decoder remains part of the network after fine-tuning.

*Hybrid Student-Teacher:* In a hybrid of the above two variants, we keep the full prediction head during fine-tuning and add a newly initialized head to predict the latent points *Q̂* directly. To distill the knowledge of the full prediction head to the latent head, we add a student-teacher-like *ℓ*₁ loss ${L}_{teach} = {\left‖\hat{Q}-stop_gradient\left({W}_{enc}\hat{P}\right)\right‖}_{1} ,$ (7) which is only backpropagated to the latent predictor, e.g., the student. During inference, the full prediction head and the frozen autoencoder acting as teacher may be removed. Instead, the frozen decoder of the student remains part of the network and we use *W*_{dec}*Q̂* as the final output, to be as lightweight as direct latent prediction.

For fine-tuning or partial re-training of the pose estimator according to all three of the above variants, mini-batch gradient descent may be used, e.g., by incorporating, say, 128 different training samples composed of sensor measurements and corresponding ground truth reference poses into a single mini-batch. Batches may be composited according to a specific scheme, such that, e.g., each training dataset is represented equally with a fixed number of examples, and/or that, e.g., training samples with ground truth 3D labels and with ground truth 2D labels are included in a mini-batch at a certain ratio, e.g., 96 3D-labeled and 32 2D-labeled samples. Ghost batch normalization, as described in the paper "Train longer, generalize better: closing the generalization gap in large batch training of neural networks" by E. Hoffer et al. may additionally be used to improve convergence in multi-dataset training, together with switching the batch normalization layers to inference mode for the last, e.g., 1000 updates. Furthermore, a smaller learning rate may be applied to the backbone than to the prediction heads for fine-tuning.

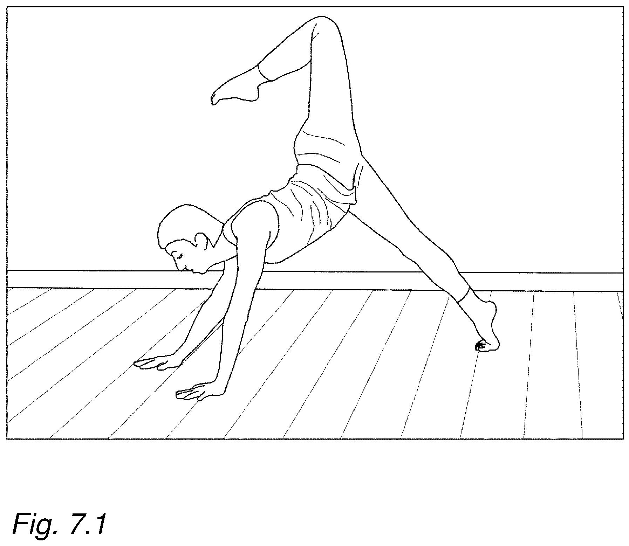

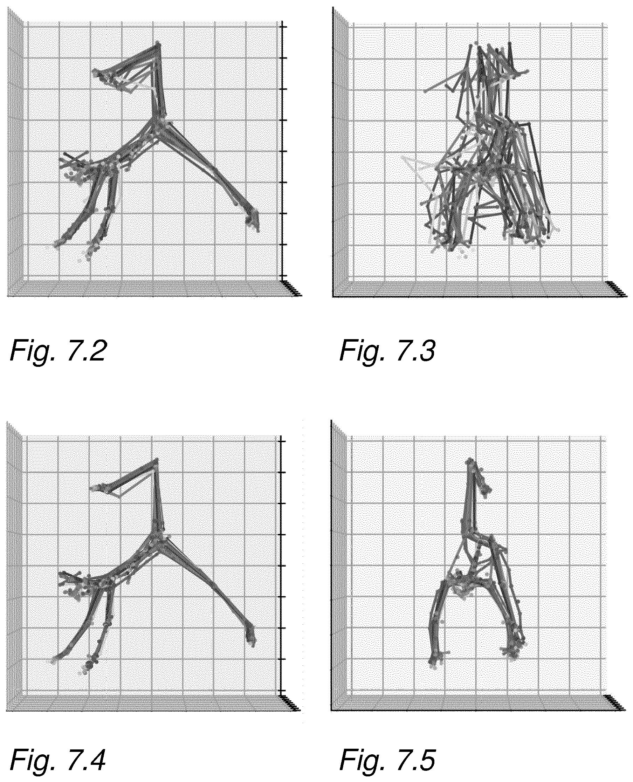

**Figure 7****.****1** schematically shows a line drawing of an example of an embodiment of a sensor measurement. The original is a color photo of a human in a particular pose. 28 different training data sets with images and 3D human poses were obtained to train 28 corresponding initial pose estimators. The 28 pose estimators were applied to the image depicted in figure 7.1, thus obtaining 28 different estimated poses for the same human. **Figure 7****.****2** shows the initial pose estimations in a front view, overlayed on top of each other. **Figure 7****.****3** shows the initial pose estimations in a side view, overlayed on top of each other. When visualizing the different pose outputs of these initial pose estimators, one sees that the pose predictions agree fairly well in the front view shown in figure 7.2. However, in the side view one sees many inconsistencies. The depth axis, e.g., the axis perpendicular to the image, is most challenging. An embodiment of a further pose estimator, following figure 6.1, was trained on all of the 28 training sets, using the affine-combining autoencoder. **Figure 7****.****4** shows the further pose estimations in a front view, overlayed on top of each other. **Figure 7****.****5** shows the further pose estimations in a side view, overlayed on top of each other. Note that in this embodiment the further pose estimator produces pose estimates in each of the available pose formats. Note that the consistency has improved, both in the front view, but especially in the side view. Note that the further pose estimator in this embodiment, produces a pose estimate in each of the pose formats, in this case using multiple prediction heads which have been fine-tuned using regularization provided by the affine-combining autoencoder.

Benchmarking embodiments against conventional pose estimators confirms that the further pose estimator indeed achieves higher accuracy. Performance on a benchmark may be measured, e.g., using the MPJPE metric. The MPJPE metric is the mean Euclidean distance between predicted and ground truth joints after alignment at the root joint. For this metric, smaller values imply more accurate results. For example, a challenging benchmark is the 3D Poses in the Wild (3DPW) dataset. See the paper 'Recovering accurate 3D human pose in the wild using IMUs and a moving camera', by T. von Marcard, et al. For this benchmark and metric, a metric of 74.7 is reported in 'Mesh Graphormer', by K. Lin, L. Wang, and Z. Liu, and a metric of 80.1 in 'Monocular, One-stage, Regression of Multiple 3D People', by Y. Sun et al. Embodiments according to figure 6.1 reach a metric of 58.9. This shows a significantly improved accuracy.

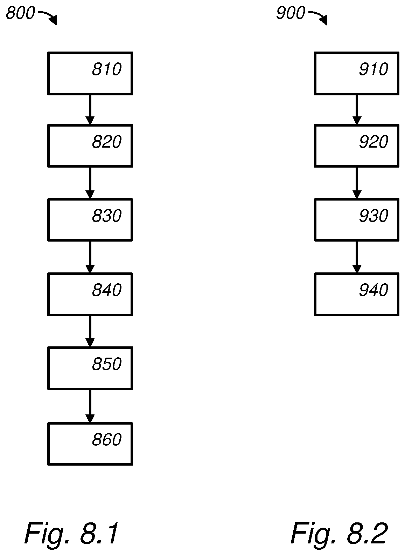

**Figure 8****.****1** schematically shows an example of an embodiment of a method (800) for training a pose estimator. The pose estimator is configured to receive as input a sensor measurement representing an object and to produce a pose, a pose comprising a plurality of key points identified in the object determining a pose of the object. Method 800 comprises
- obtaining (810) multiple training data sets, a training data set comprising multiple pairs of a sensor measurement and a pose, different training data sets of the multiple training data sets using a different pose format,
- obtaining (820) multiple trained initial pose estimators for the multiple training data sets, a trained initial pose estimator of the multiple trained initial pose estimators being trained on a corresponding training data set of the multiple training data sets,
- selecting (830) a pool of sensor measurements representing an object and applying (840) the multiple trained initial pose estimators to the pool, thus obtaining multiple estimated poses for a sensor measurement in the pool,
- training (850) an autoencoder on the multiple estimated poses, the autoencoder being configured to receive at least one pose according to a pose format corresponding to a first training data set and to produce at least one pose according to a pose format corresponding to a second training data set,
- training (860) a further pose estimator on more than one of the multiple training data sets using at least part of the trained autoencoder to map a pose from a pose format corresponding to a first training data set to a pose format corresponding to a second training data set.

**Figure 8****.****2** schematically shows an example of an embodiment of a method (900) for controlling a robotic system. Method 900 comprises
- obtaining (910) a further pose estimator. For example, obtaining the further pose estimator may comprise training the further pose estimator, e.g., may comprise method 800. The further pose estimator may also be obtained from another source, e.g., from a network, a storage, or the like.
- receiving (920) a sensor measurement from a sensor,
- applying (930) the further pose estimator to the sensor measurement and obtaining a pose of an object represented in the sensor measurement,
- deriving (940) a control signal from at least the pose, and controlling the robotic system with the control signal.

For example, training method 800 and control method 900 may be computer implemented methods. For example, accessing training data, and/or receiving input data may be done using a communication interface, e.g., an electronic interface, a network interface, a memory interface, etc. For example, storing or retrieving parameters may be done from an electronic storage, e.g., a memory, a hard drive, etc., e.g., parameters of the networks. For example, applying a neural network to data of the training data, and/or adjusting the stored parameters to train the network may be done using an electronic computing device, e.g., a computer. A pose estimator, and/or autoencoder may also output mean and/or variance, instead of directly the output. The neural networks, either during training and/or during inference may have multiple layers, which may include, e.g., convolutional layers and the like. For example, the neural network may have at least 2, 5, 10, 15, 20 or 40 hidden layers, or more, etc. The number of neurons in the neural network may, e.g., be at least 10, 100, 1000, 10000, 100000, 1000000, or more, etc.

Many different ways of executing the method are possible, as will be apparent to a person skilled in the art. For example, the order of the steps can be performed in the shown order, but the order of the steps can be varied or some steps may be executed in parallel. Moreover, in between steps other method steps may be inserted. The inserted steps may represent refinements of the method such as described herein, or may be unrelated to the method. For example, some steps may be executed, at least partially, in parallel. Moreover, a given step may not have finished completely before a next step is started.

Embodiments of the method may be executed using software, which comprises instructions for causing a processor system to perform method 800 and/or 900. Software may only include those steps taken by a particular sub-entity of the system. The software may be stored in a suitable storage medium, such as a hard disk, a floppy, a memory, an optical disc, etc. The software may be sent as a signal along a wire, or wireless, or using a data network, e.g., the Internet. The software may be made available for download and/or for remote usage on a server. Embodiments of the method may be executed using a bitstream arranged to configure programmable logic, e.g., a field-programmable gate array (FPGA), to perform the method.

It will be appreciated that the presently disclosed subject matter also extends to computer programs, particularly computer programs on or in a carrier, adapted for putting the presently disclosed subject matter into practice. The program may be in the form of source code, object code, a code intermediate source, and object code such as partially compiled form, or in any other form suitable for use in the implementation of an embodiment of the method. An embodiment relating to a computer program product comprises computer executable instructions corresponding to each of the processing steps of at least one of the methods set forth. These instructions may be subdivided into subroutines and/or be stored in one or more files that may be linked statically or dynamically. Another embodiment relating to a computer program product comprises computer executable instructions corresponding to each of the devices, units and/or parts of at least one of the systems and/or products set forth.

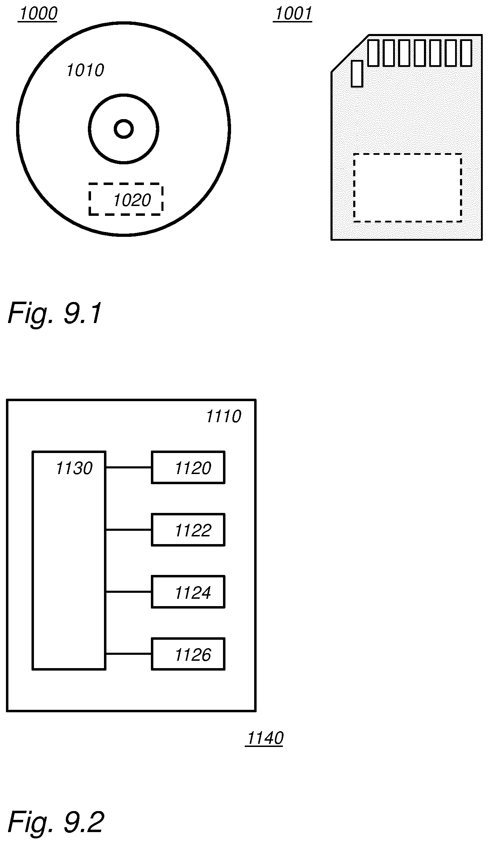

**Figure 9****.****1** shows a computer readable medium 1000 having a writable part 1010, and a computer readable medium 1001 also having a writable part. Computer readable medium 1000 is shown in the form of an optically readable medium. Computer readable medium 1001 is shown in the form of an electronic memory, in this case a memory card. Computer readable medium 1000 and 1001 may store data 1020 wherein the data may indicate instructions, which when executed by a processor system, cause a processor system to perform an embodiment of a training and/or controlling method, according to an embodiment. The computer program 1020 may be embodied on the computer readable medium 1000 as physical marks or by magnetization of the computer readable medium 1000. However, any other suitable embodiment is conceivable as well. Furthermore, it will be appreciated that, although the computer readable medium 1000 is shown here as an optical disc, the computer readable medium 1000 may be any suitable computer readable medium, such as a hard disk, solid state memory, flash memory, etc., and may be non-recordable or recordable. The computer program 1020 comprises instructions for causing a processor system to perform said a training and/or controlling method.

**Figure 9****.****2** shows in a schematic representation of a processor system 1140 according to an embodiment of a training and/or controlling system. The processor system comprises one or more integrated circuits 1110. The architecture of the one or more integrated circuits 1110 is schematically shown in Figure 9.2. Circuit 1110 comprises a processing unit 1120, e.g., a CPU, for running computer program components to execute a method according to an embodiment and/or implement its modules or units. Circuit 1110 comprises a memory 1122 for storing programming code, data, etc. Part of memory 1122 may be read-only. Circuit 1110 may comprise a communication element 1126, e.g., an antenna, connectors or both, and the like. Circuit 1110 may comprise a dedicated integrated circuit 1124 for performing part or all of the processing defined in the method. Processor 1120, memory 1122, dedicated IC 1124 and communication element 1126 may be connected to each other via an interconnect 1130, say a bus. The processor system 1110 may be arranged for contact and/or contact-less communication, using an antenna and/or connectors, respectively.

For example, in an embodiment, processor system 1140, e.g., the training and/or controlling system or device may comprise a processor circuit and a memory circuit, the processor being arranged to execute software stored in the memory circuit. For example, the processor circuit may be an Intel Core i7 processor, ARM Cortex-R8, etc. The memory circuit may be an ROM circuit, or a non-volatile memory, e.g., a flash memory. The memory circuit may be a volatile memory, e.g., an SRAM memory. In the latter case, the device may comprise a non-volatile software interface, e.g., a hard drive, a network interface, etc., arranged for providing the software.

It will be apparent that various information described as stored in the storage 1122. Various other arrangements will be apparent. Further, the memory 1122 may be considered to be "non-transitory machine-readable media." As used herein, the term "non-transitory" will be understood to exclude transitory signals but to include all forms of storage, including both volatile and non-volatile memories.

While device 1100 is shown as including one of each described component, the various components may be duplicated in various embodiments. For example, the processor 1120 may include multiple microprocessors that are configured to independently execute the methods described herein or are configured to perform steps or subroutines of the methods described herein such that the multiple processors cooperate to achieve the functionality described herein. Further, where the device 1100 is implemented in a cloud computing system, the various hardware components may belong to separate physical systems. For example, the processor 1120 may include a first processor in a first server and a second processor in a second server.

In the claims, any reference signs placed between parentheses shall not be construed as limiting the claim. Use of the verb 'comprise' and its conjugations does not exclude the presence of elements or steps other than those stated in a claim. The article 'a' or 'an' preceding an element does not exclude the presence of a plurality of such elements. Expressions such as "at least one of" when preceding a list of elements represent a selection of all or of any subset of elements from the list. For example, the expression, "at least one of A, B, and C" should be understood as including only A, only B, only C, both A and B, both A and C, both B and C, or all of A, B, and C. The presently disclosed subject matter may be implemented by hardware comprising several distinct elements, and by a suitably programmed computer. In the device claim enumerating several parts, several of these parts may be embodied by one and the same item of hardware. The mere fact that certain measures are recited in mutually different dependent claims does not indicate that a combination of these measures cannot be used to advantage.

In the claims references in parentheses refer to reference signs in drawings of exemplifying embodiments or to formulas of embodiments, thus increasing the intelligibility of the claim. These references shall not be construed as limiting the claim.

## Claims

1. A computer-implemented method (800) for training a pose estimator, the pose estimator being configured to receive as input a sensor measurement representing an object and to produce a pose of the object, wherein a pose comprises a plurality of key points identified in the object according to a pose format, which key points determine the pose of the object, a pose format for a pose defining the key points included in the pose and the order of the key points in the pose,
- obtaining (810) multiple training data sets, a training data set comprising multiple pairs of a sensor measurement and a pose, different training data sets of the multiple training data sets using a different pose format,
- obtaining (820) multiple trained initial pose estimators for the multiple training data sets, a trained initial pose estimator of the multiple trained initial pose estimators being trained on a corresponding training data set of the multiple training data sets,
- selecting (830) a pool of sensor measurements representing an object and applying (840) the multiple trained initial pose estimators to the pool, thus obtaining multiple estimated poses for a sensor measurement in the pool,
- training (850) an autoencoder on the multiple estimated poses, the autoencoder being configured to receive at least one pose according to a pose format corresponding to a first training data set and to produce at least one pose according to a pose format corresponding to a second training data set,
- training (860) a further pose estimator on more than one of the multiple training data sets using at least part of the trained autoencoder to map a pose from a pose format corresponding to a first training data set to a pose format corresponding to a second training data set.

2. A method as in Claim 1, wherein the object is one or more of: an articulated object, a non-articulated object, an animal, a human, a vehicle, a car, a bicycle, a pallet truck, a forklift.

3. A method as in any one of the preceding claims, wherein
- the multiple trained initial pose estimators comprise a shared-backbone and multiple prediction heads, the shared-backbone being configured to receive the sensor measurement, a prediction head of the multiple prediction heads corresponding to the pose format of a training data set of the multiple training data sets and being configured to receive the output of the shared-backbone and to produce the estimated pose for the sensor measurement according to said pose format, and/or
- the multiple trained initial pose estimators comprising multiple individual models corresponding to multiple pose formats of the multiple training data sets.

4. A method as in any one of the preceding claims, wherein
- a training data set comprises a sequence of sensor measurements, the method comprising determining a deviation between poses in the training data set for sensor measurements in the sequence, and selecting for the pool a sensor measurement having a deviation below a threshold, and/or
- the method comprises determining an occlusion of the object, and selecting for the pool a sensor measurement having an occlusion below a threshold, and/or,
- the method comprises obtaining an accuracy for poses in a training data set, and selecting for the pool a sensor measurement having an accuracy above a threshold.

5. A method as in any one of the preceding claims, wherein the autoencoder is configured to receive as input multiple poses for the same sensor measurement according to the pose format of multiple training data sets, the autoencoder comprising
- an encoder configured to receive as input the multiple poses, and to produce a representation in a latent space, and
- a decoder configured to receive as input the representation in the latent space, and to produce the multiple poses as output.

6. A method as in any one of the preceding claims, wherein the autoencoder is configured to receive as input multiple poses for the same sensor measurement according to the pose format of multiple training data sets, wherein
- the autoencoder is configured to compute points in a latent space as combinations of points in the input poses, and/or
- the decoder is configured to compute a point in the output as combination of points in the latent space.

7. A method as in any one of the preceding claims, wherein the object has a chirality equivalence, training the autoencoder being subject to one or more equality conditions on parameters of the autoencoder ensuring chirality equivariance.

8. A method as in any one of the preceding claims, wherein the further pose estimator is configured to receive as input a sensor measurement and to produce as output a pose according to the multiple pose formats corresponding to the multiple training data sets, the further pose estimator being trained on the multiple training data sets, said training comprising minimizing a combination of one or more losses, the one or more losses comprising a consistency loss indicating a consistency of the multiple pose formats according to the trained autoencoder.

9. A method as in any one of the preceding claims, wherein the further pose estimator is configured to receive as input a sensor measurement, the further pose estimator being trained on the multiple training data sets, said training comprising minimizing a combination of one or more losses, the one or more losses comprising one or more pose losses, obtained from comparing a training pose in the multiple training data sets corresponding to a training sensor measurement to a corresponding output pose of the further pose estimator and/or of the autoencoder applied to the output of the further pose estimator.

10. A method as in any one of the preceding claims, wherein the further pose estimator is configured to receive as input a sensor measurement and to produce an output in a latent space of the autoencoder,
- training the further pose estimator comprises applying the further pose estimator to a training sensor measurement from a training data set of the multiple training data sets, obtaining from the output of the further pose estimator, using at least part of the autoencoder, a transformed pose according to the pose format of the training data set, and minimizing a pose loss obtained from comparing the transformed pose and the training pose corresponding to the training sensor measurement.

11. A method as in Claim 10, wherein a first further pose estimator configured to produce an output in a latent space of the autoencoder is trained from a second further pose estimator configured to produce multiple pose formats.

12. A method for controlling a robotic system,
- training a further pose estimator according to any one of the methods of Claims 1-11,
- receiving a sensor measurement from a sensor,
- applying the further pose estimator to the sensor measurement and obtaining a pose, obtaining a pose of an object represented in the sensor measurement,
- deriving a control signal from at least the pose, and controlling the robotic system with the control signal.

13. A method as in Claim 12, wherein
- the robotic system is configured to manipulate the object represented in the sensor measurement, the control signal being configured to manipulate the object, and/or
- the robotic system is an autonomous vehicle, the control signal being configured to avoid a collision with the object, the object comprising one or more of: a human, another vehicle, a static obstacle.

14. A system comprising: one or more processors; and one or more storage devices storing instructions that, when executed by the one or more processors, cause the one or more processors to perform operations for a method according to any one of the preceding claims.

15. A transitory or non-transitory computer storage medium encoded with instructions that, when executed by one or more processors, cause the one or more processors to perform operations according to any one of the preceding claims.

## Patentansprüche

1. Computerimplementiertes Verfahren (800) zum Trainieren eines Posenschätzers, wobei der Posenschätzer dazu ausgelegt ist, als Eingabe eine Sensormessung zu empfangen, die ein Objekt darstellt, und eine Pose des Objekts zu erzeugen, wobei eine Pose eine Mehrzahl von Schlüsselpunkten umfasst, die in dem Objekt gemäß einem Posenformat identifiziert werden, wobei die Schlüsselpunkte die Pose des Objekts bestimmen, wobei ein Posenformat für eine Pose die in der Pose enthaltenen Schlüsselpunkte und die Reihenfolge der Schlüsselpunkte in der Pose definiert,
- Erhalten (810) mehrerer Trainingsdatensätze, wobei ein Trainingsdatensatz mehrere Paare aus einer Sensormessung und einer Pose umfasst, wobei unterschiedliche Trainingsdatensätze der mehreren Trainingsdatensätze ein unterschiedliches Posenformat verwenden,
- Erhalten (820) mehrerer trainierter anfänglicher Posenschätzer für die mehreren Trainingsdatensätze, wobei ein trainierter anfänglicher Posenschätzer der mehreren trainierten anfänglichen Posenschätzer auf einem entsprechenden Trainingsdatensatz der mehreren Trainingsdatensätze trainiert wird,
- Auswählen (830) eines Pools von Sensormessungen, die ein Objekt darstellen, und Anwenden (840) der mehreren trainierten anfänglichen Posenschätzer auf den Pool, wodurch mehrere geschätzte Posen für eine Sensormessung in dem Pool erhalten werden,
- Trainieren (850) eines Autoencoders auf den mehreren geschätzten Posen, wobei der Autoencoder dazu ausgelegt ist, mindestens eine Pose gemäß einem Posenformat zu empfangen, das einem ersten Trainingsdatensatz entspricht, und mindestens eine Pose gemäß einem Posenformat zu erzeugen, das einem zweiten Trainingsdatensatz entspricht,
- Trainieren (860) eines weiteren Posenschätzers auf mehr als einem der mehreren Trainingsdatensätze unter Verwendung mindestens eines Teils des trainierten Autoencoders, um eine Pose von einem Posenformat, das einem ersten Trainingsdatensatz entspricht, auf ein Posenformat abzubilden, das einem zweiten Trainingsdatensatz entspricht.

2. Verfahren nach Anspruch 1, wobei das Objekt eines oder mehrere von Folgendem ist: ein gelenkiges Objekt, ein nicht gelenkiges Objekt, ein Tier, ein Mensch, ein Fahrzeug, ein Pkw, ein Fahrrad, ein Hubwagen, ein Gabelstapler.

3. Verfahren nach einem der vorhergehenden Ansprüche, wobei
- die mehreren trainierten anfänglichen Posenschätzer ein gemeinsam genutztes Backbone und mehrere Vorhersageköpfe umfassen, wobei das gemeinsam genutzte Backbone dazu ausgelegt ist, die Sensormessung zu empfangen, wobei ein Vorhersagekopf der mehreren Vorhersageköpfe dem Posenformat eines Trainingsdatensatzes der mehreren Trainingsdatensätze entspricht und dazu ausgelegt ist, die Ausgabe des gemeinsam genutzten Backbones zu empfangen und die geschätzte Pose für die Sensormessung gemäß dem Posenformat zu erzeugen, und/oder
- wobei die mehreren trainierten anfänglichen Posenschätzer mehrere individuelle Modelle umfassen, die mehreren Posenformaten der mehreren Trainingsdatensätze entsprechen.

4. Verfahren nach einem der vorhergehenden Ansprüche, wobei
- ein Trainingsdatensatz eine Sequenz von Sensormessungen umfasst, wobei das Verfahren Bestimmen einer Abweichung zwischen Posen in dem Trainingsdatensatz für Sensormessungen in der Sequenz und Auswählen einer Sensormessung mit einer Abweichung unterhalb eines Schwellenwerts für den Pool umfasst, und/oder
- das Verfahren Bestimmen einer Verdeckung des Objekts und Auswählen einer Sensormessung mit einer Verdeckung unterhalb eines Schwellenwerts für den Pool umfasst, und/oder
- das Verfahren Erhalten einer Genauigkeit für Posen in einem Trainingsdatensatz und Auswählen einer Sensormessung mit einer Genauigkeit oberhalb eines Schwellenwerts für den Pool umfasst.

5. Verfahren nach einem der vorhergehenden Ansprüche, wobei der Autoencoder dazu ausgelegt ist, als Eingabe mehrere Posen für die gleiche Sensormessung gemäß dem Posenformat mehrerer Trainingsdatensätze zu empfangen, wobei der Autoencoder Folgendes umfasst
- einen Encoder, der dazu ausgelegt ist, als Eingabe die mehreren Posen zu empfangen und eine Darstellung in einem latenten Raum zu erzeugen, und
- einen Decoder, der dazu ausgelegt ist, als Eingabe die Darstellung in dem latenten Raum zu empfangen und die mehreren Posen als Ausgabe zu erzeugen.

6. Verfahren nach einem der vorhergehenden Ansprüche, wobei der Autoencoder dazu ausgelegt ist, als Eingabe mehrere Posen für die gleiche Sensormessung gemäß dem Posenformat mehrerer Trainingsdatensätze zu empfangen, wobei
- der Autoencoder dazu ausgelegt ist, Punkte in einem latenten Raum als Kombinationen von Punkten in den eingegebenen Posen zu berechnen, und/oder
- der Decoder dazu ausgelegt ist, einen Punkt in der Ausgabe als Kombination von Punkten in dem latenten Raum zu berechnen.

7. Verfahren nach einem der vorhergehenden Ansprüche, wobei das Objekt eine Chiralitätsäquivalenz aufweist, wobei das Trainieren des Autoencoders einer oder mehreren Gleichheitsbedingungen an Parametern des Autoencoders unterliegt, die eine Chiralitätsäquivarianz sicherstellen.

8. Verfahren nach einem der vorhergehenden Ansprüche, wobei der weitere Posenschätzer dazu ausgelegt ist, als Eingabe eine Sensormessung zu empfangen und als Ausgabe eine Pose gemäß den mehreren Posenformaten zu erzeugen, die den mehreren Trainingsdatensätzen entsprechen, wobei der weitere Posenschätzer auf den mehreren Trainingsdatensätzen trainiert wird, wobei das Training Minimieren einer Kombination von einem oder mehreren Verlusten umfasst, wobei der eine oder die mehreren Verluste einen Konsistenzverlust umfassen, der eine Konsistenz der mehreren Posenformate gemäß dem trainierten Autoencoder angibt.

9. Verfahren nach einem der vorhergehenden Ansprüche, wobei der weitere Posenschätzer dazu ausgelegt ist, als Eingabe eine Sensormessung zu empfangen, wobei der weitere Posenschätzer auf den mehreren Trainingsdatensätzen trainiert wird, wobei das Training Minimieren einer Kombination von einem oder mehreren Verlusten umfasst, wobei der eine oder die mehreren Verluste einen oder mehrere Posenverluste umfassen, die aus einem Vergleichen einer Trainingspose in den mehreren Trainingsdatensätzen, die einer Trainingssensormessung entsprechen, mit einer entsprechenden ausgegebenen Pose des weiteren Posenschätzers und/oder des Autoencoders erhalten werden, die auf die Ausgabe des weiteren Posenschätzers angewendet wird.

10. Verfahren nach einem der vorhergehenden Ansprüche, wobei der weitere Posenschätzer dazu ausgelegt ist, als Eingabe eine Sensormessung zu empfangen und eine Ausgabe in einem latenten Raum des Autoencoders zu erzeugen,
- wobei das Trainieren des weiteren Posenschätzers Anwenden des weiteren Posenschätzers auf eine Trainingssensormessung aus einem Trainingsdatensatz der mehreren Trainingsdatensätze, Erhalten einer transformierten Pose gemäß dem Posenformat des Trainingsdatensatzes aus der Ausgabe des weiteren Posenschätzers unter Verwendung mindestens eines Teils des Autoencoders und Minimieren eines Posenverlusts, der aus einem Vergleichen der transformierten Pose und der Trainingspose, die der Trainingssensormessung entspricht, erhalten wird, umfasst.

11. Verfahren nach Anspruch 10, wobei ein erster weiterer Posenschätzer, der dazu ausgelegt ist, eine Ausgabe in einem latenten Raum des Autoencoders zu erzeugen, von einem zweiten weiteren Posenschätzer trainiert wird, der dazu ausgelegt ist, mehrere Posenformate zu erzeugen.

12. Verfahren zum Steuern eines Robotersystems,
- Trainieren eines weiteren Posenschätzers gemäß einem der Verfahren nach den Ansprüchen 1-11,
- Empfangen einer Sensormessung von einem Sensor,
- Anwenden des weiteren Posenschätzers auf die Sensormessung und Erhalten einer Pose, Erhalten einer Pose eines in der Sensormessung dargestellten Objekts,
- Ableiten eines Steuersignals aus mindestens der Pose und Steuern des Robotersystems mit dem Steuersignal.

13. Verfahren nach Anspruch 12, wobei
- das Robotersystem dazu ausgelegt ist, das in der Sensormessung dargestellte Objekt zu manipulieren, wobei das Steuersignal dazu ausgelegt ist, das Objekt zu manipulieren, und/oder
- das Robotersystem ein autonomes Fahrzeug ist, wobei das Steuersignal dazu ausgelegt ist, eine Kollision mit dem Objekt zu vermeiden, wobei das Objekt eines oder mehrere von Folgendem umfasst: einen Menschen, ein anderes Fahrzeug, ein statisches Hindernis.

14. System, das Folgendes umfasst: einen oder mehrere Prozessoren; und eine oder mehrere Speichervorrichtungen, die Anweisungen speichern, die, wenn sie durch den einen oder die mehreren Prozessoren ausgeführt werden, bewirken, dass der eine oder die mehreren Prozessoren Operationen für ein Verfahren nach einem der vorhergehenden Ansprüche durchführen.

15. Flüchtiges oder nichtflüchtiges Computerspeichermedium, das mit Anweisungen codiert ist, die, wenn sie durch einen oder mehrere Prozessoren ausgeführt werden, bewirken, dass der eine oder die mehreren Prozessoren Operationen nach einem der vorhergehenden Ansprüche durchführen.

## Revendications

1. Procédé, (800) mis en œuvre par ordinateur, d'entraînement d'un estimateur de pose, l'estimateur de pose étant configuré pour recevoir en entrée une mesure de capteur représentant un objet et pour produire une pose de l'objet, une pose comprenant une pluralité de points clés identifiés dans l'objet selon un format de pose, lesdits points clés déterminant la pose de l'objet, un format de pose pour une pose définissant les points clés inclus dans la pose et l'ordre des points clés dans la pose, comprenant
- l'obtention (810) de multiples ensembles de données d'apprentissage, un ensemble de données d'apprentissage comprenant de multiples couples d'une mesure de capteur et d'une pose, des ensembles de données d'apprentissage différents parmi les multiples ensembles de données d'apprentissage utilisant un format de pose différent,
- l'obtention (820) de multiples estimateurs de pose initiale entraînés pour les multiples ensembles de données d'apprentissage, un estimateur de pose initiale entraîné parmi les multiples estimateurs de pose initiale entraînés étant entraîné sur un ensemble de données d'apprentissage correspondant parmi les multiples ensembles de données d'apprentissage,
- la sélection (830) d'un groupe de mesures de capteurs représentant un objet et l'application (840) des multiples estimateurs de pose initiale entraînés au groupe, obtenant ainsi de multiples poses estimées pour une mesure de capteurs dans le groupe,
- l'entraînement (850) d'un auto-encodeur sur les multiples poses estimées, l'auto-encodeur étant configuré pour recevoir au moins une pose selon un format de pose correspondant à un premier ensemble de données d'apprentissage et pour produire au moins une pose selon un format de pose correspondant à un second ensemble de données d'apprentissage,
- l'entraînement (860) d'un estimateur de pose supplémentaire sur plus d'un des multiples ensembles de données d'apprentissage en utilisant au moins une partie de l'auto-encodeur entraîné pour transposer une pose d'un format de pose correspondant à un premier ensemble de données d'apprentissage vers un format de pose correspondant à un second ensemble de données d'apprentissage.

2. Procédé selon la revendication 1, l'objet étant un ou plusieurs objets parmi : un objet articulé, un objet non articulé, un animal, un être humain, un véhicule, une voiture, un vélo, un transpalette, un chariot élévateur.

3. Procédé selon l'une quelconque des revendications précédentes,
- les multiples estimateurs de pose initiale entraînés comportant une colonne vertébrale partagée et de multiples têtes de prédiction, la colonne vertébrale partagée étant configurée pour recevoir la mesure de capteur, une tête de prédiction parmi les multiples têtes de prédiction correspondant au format de pose d'un ensemble de données d'apprentissage parmi les multiples ensembles de données d'apprentissage et étant configurée pour recevoir la sortie de la colonne vertébrale partagée et pour produire la pose estimée pour la mesure de capteur selon ledit format de pose, et/ou
- les multiples estimateurs de pose initiale entraînés comprenant de multiples modèles individuels correspondant à de multiples formats de pose des multiples ensembles de données d'apprentissage.

4. Procédé selon l'une quelconque des revendications précédentes,
- un ensemble de données d'apprentissage comprenant une séquence de mesures de capteur, le procédé comprenant la détermination d'un écart entre des poses dans l'ensemble de données d'apprentissage pour des mesures de capteur dans la séquence, et la sélection pour le groupe d'une mesure de capteur présentant un écart inférieur à un seuil, et/ou
- le procédé comprenant la détermination d'une occultation de l'objet, et la sélection pour le groupe d'une mesure de capteur présentant une occultation inférieure à un seuil, et/ou,
- le procédé comprenant l'obtention d'une précision pour des poses dans un ensemble de données d'apprentissage, et la sélection pour le groupe d'une mesure de capteur présentant une précision supérieure à un seuil.

5. Procédé selon l'une quelconque des revendications précédentes, l'auto-encodeur étant configuré pour recevoir en entrée de multiples poses pour la même mesure de capteur selon le format de pose de multiples ensembles de données d'apprentissage, l'auto-encodeur comprenant
- un encodeur configuré pour recevoir en entrée les multiples poses, et pour réaliser une représentation dans un espace latent, et
- un décodeur configuré pour recevoir en entrée la représentation dans l'espace latent, et pour produire en sortie les multiples poses.

6. Procédé selon l'une quelconque des revendications précédentes, l'auto-encodeur étant configuré pour recevoir en entrée de multiples poses pour la même mesure de capteur selon le format de pose de multiples ensembles de données d'apprentissage,
- l'auto-encodeur étant configuré pour calculer des points dans un espace latent comme des combinaisons de points dans les poses d'entrée, et/ou
- le décodeur étant configuré pour calculer un point dans la sortie comme combinaison de points dans l'espace latent.

7. Procédé selon l'une quelconque des revendications précédentes, l'objet présentant une équivalence de chiralité, l'entraînement de l'auto-encodeur étant soumis à une ou plusieurs conditions d'égalité sur des paramètres de l'auto-encodeur assurant l'équivariance de chiralité.

8. Procédé selon l'une quelconque des revendications précédentes, l'estimateur de pose supplémentaire étant configuré pour recevoir en entrée une mesure de capteur et pour produire en sortie une pose selon les multiples formats de pose correspondant aux multiples ensembles de données d'apprentissage, l'estimateur de pose supplémentaire étant entraîné sur les multiples ensembles de données d'apprentissage, ledit entraînement comprenant la minimisation d'une combinaison d'une ou plusieurs pertes, la ou les pertes comprenant une perte de cohérence indiquant une cohérence des multiples formats de pose selon l'auto-encodeur entraîné.

9. Procédé selon l'une quelconque des revendications précédentes, l'estimateur de pose supplémentaire étant configuré pour recevoir en entrée une mesure de capteur, l'estimateur de pose supplémentaire étant entraîné sur les multiples ensembles de données d'apprentissage, ledit apprentissage comprenant la minimisation d'une combinaison d'une ou plusieurs pertes, la ou les pertes comprenant une ou plusieurs pertes de pose, obtenues en comparant une pose d'apprentissage dans les multiples ensembles de données d'apprentissage correspondant à une mesure de capteur d'apprentissage à une pose de sortie correspondante de l'estimateur de pose supplémentaire et/ou de l'auto-encodeur appliqué à la sortie de l'estimateur de pose supplémentaire.

10. Procédé selon l'une quelconque des revendications précédentes, l'estimateur de pose supplémentaire étant configuré pour recevoir en entrée une mesure de capteur et pour produire une sortie dans un espace latent de l'auto-encodeur.
- l'apprentissage de l'estimateur de pose supplémentaire comprenant l'application de l'estimateur de pose supplémentaire à une mesure de capteur d'apprentissage issue d'un ensemble de données d'apprentissage parmi les multiples ensembles de données d'apprentissage, l'obtention, à partir de la sortie de l'estimateur de pose supplémentaire, en utilisant au moins une partie de l'auto-encodeur, d'une pose transformée selon le format de pose de l'ensemble de données d'apprentissage, et la minimisation d'une perte de pose obtenue à partir de la comparaison de la pose transformée et de la pose d'apprentissage correspondant à la mesure de capteur d'apprentissage.

11. Procédé selon la revendication 10, un premier estimateur de pose supplémentaire configuré pour produire une sortie dans un espace latent de l'auto-encodeur étant entraîné à partir d'un second estimateur de pose supplémentaire configuré pour produire de multiples formats de pose.

12. Procédé de commande d'un système robotique, comprenant
- l'entraînement d'un estimateur de pose supplémentaire selon l'un quelconque des procédés des revendications 1 à 11,
- la réception d'une mesure de capteur provenant d'un capteur,
- l'application de l'estimateur supplémentaire de pose à la mesure de capteur et l'obtention d'une pose, obtenant une pose d'un objet représenté dans la mesure de capteur,
- l'élaboration d'un signal de commande à partir d'au moins la pose, et la commande du système robotique avec le signal de commande.

13. Procédé selon la revendication 12,
- le système robotique étant configuré pour manipuler l'objet représenté dans la mesure de capteur, le signal de commande étant configuré pour manipuler l'objet, et/ou
- le système robotique étant un véhicule autonome, le signal de commande étant configuré pour éviter une collision avec l'objet, l'objet comprenant un ou plusieurs objets parmi : un humain, un autre véhicule, un obstacle statique.

14. Système comprenant : un ou plusieurs processeurs ; et un ou plusieurs dispositifs de stockage stockant des instructions qui, lorsqu'elles sont exécutées par le ou les processeurs, amènent le ou les processeurs à effectuer des opérations pour un procédé selon l'une quelconque des revendications précédentes.

15. Support de stockage informatique transitoire ou non transitoire codé avec des instructions qui, lorsqu'elles sont exécutées par un ou plusieurs processeurs, amènent le ou les processeurs à effectuer des opérations selon l'une quelconque des revendications précédentes.
